# EUROPEAN PATENT SPECIFICATION

(11) **EP 3 976 013 B1**
(45) Date of publication and mention of the grant of the patent: **28.10.2026**
(21) Application number: 20813551.7
(22) Date of filing: 29.05.2020
(51) Int. Cl.: A61K 31/135, A61P 29/00, A61P 37/02, A61P 25/00, A61K 45/06, A61K 31/216, A61K 31/567, A61P 25/02, A61P 25/06, A61P 25/22, A61P 25/24, A61P 25/28

(54) **AMITRIPTYLINE FOR USE IN THE TREATMENT OF PREMENSTRUAL SYNDROME (PMS) OR PREMENSTRUAL DYSPHORIC DISORDER (PMDD)**
AMITRIPTYLIN ZUR VERWENDUNG BEI DER BEHANDLUNG DES PRÄMENSTRUELLEN SYNDROMS (PMS) ODER DER PRÄMENSTRUELLEN DYSPHORISCHEN STÖRUNG (PMDS)
AMITRIPTYLINE POUR UTILISATION DANS LE TRAITEMENT DU SYNDROME PRÉMENSTRUEL (SPM) OU DU TROUBLE DYSPHORIQUE PRÉMENSTRUEL (TDPM)

(30) Priority: 31.05.2019 AU 2019901883
(43) Date of publication of application: 06.04.2022
(73) Proprietor: Alyra Biotech Pty Ltd, Wayville, SA 5034 (AU)
(72) Inventor: EVANS, Susan, Florence, Kent Town, South Australia 5067 (AU)
(74) Representative: Schnappauf, Georg
(86) International application number: PCT/AU2020/050551
(87) International publication number: WO 2020/237325

(56) References cited:
- WO-A1-2014/121332
- WO-A1-2019/119059
- WO-A2-2008/076804
- CA-C- 2 002 182
- BALDASZTI ERIKA ET AL: "Acceptability of the long-term contraceptive levonorgestrel-releasing intrauterine system (Mirena®): a 3-year follow-up study", CONTRACEPTION., vol. 67, no. 2, 1 February 2003 (2003-02-01), US, pages 87 - 91, XP055837251, ISSN: 0010-7824, DOI: 10.1016/S0010-7824(02)00482-1
- LIANG NA ET AL: "Levonorgestrel Ameliorates Adenomyosis via lncRNA H19/miR-17/TLR4 Pathway", vol. Volume 14, 1 January 2020 (2020-01-01), pages 3449 - 3460, XP055837057, Retrieved from the Internet <URL:https://pdfs.semanticscholar.org/7662/1c8318cf839af083e233c672153f8dfa42b6.pdf?_ga=2.172047523.1633636701.1630570643-2068618810.1630570643> DOI: 10.2147/DDDT.S248095
- BEHR DAVIS ET AL: "The effects of premenstrual syndrome (PMS) on the female singer", JOURNAL OF VOICE, ELSEVIER SCIENCE, US, vol. 7, no. 4, 1 December 2005 (2005-12-01), pages 337 - 353, XP005312875, ISSN: 0892-1997
- DORDUNOO, SK ET AL.: "Pharmacokinetics and biodistribution of paclitaxel following intrauterine administration of PACLIMER microspheres in female New Zealand rabbits", PROCEEDINGS OF THE AMERICAN ASSOCIATION OF CANCER RESEARCH ANNUAL MEET, vol. 47, 2006, XP055763457

## Description

### FIELD

The present disclosure relates, at least in part, to methods, compositions and devices for treating neuroinflammatory conditions in female subjects.

### BACKGROUND

Neuroinflammation plays a role in a variety of conditions of the central nervous system and the peripheral nervous system. Indeed, it is now recognised that inflammatory processes contribute to many neurological disorders, such as multiple sclerosis, migraine, ageing-related neurodegeneration, neuropsychiatric conditions, pain and the perception of pain. There is also evidence to suggest that the aetiology of some neuropsychiatric disorders in women associated with the menstrual cycle, such as premenstrual dysphoric syndrome (PMDDS) and premenstrual syndrome (PMS), involve neuroinflammation.

Neuroinflammation involves the biochemical and cellular response of the nervous system to injury, infection, autoimmunity or neurodegenerative conditions and involves the activation of glia and the release of inflammatory mediators. Upon exposure to certain stimuli in the periphery, glial cells including astrocytes and microglia within the central nervous system respond by upregulation of inflammatory signals that lead to neuroinflammation. The recently discovered link between the brain and the body, the lymphatic vessels of the brain, provides an additional mechanism whereby peripheral inflammation influences central nervous system function

Treatments for neuroinflammatory conditions are varied and complex. An increasing number of treatments aim to reduce inflammation via use of anti-inflammatory agents, antioxidants, steroids, neuroimmune treatments including monoclonal antibodies, and nutritional supplements. However, current therapies vary in their efficacy and in some cases possess significant side effects.

International Patent Application Publication No. WO 2019/119059 A1 discloses methods, compositions and products for treating pain, and/or pain related symptoms, associated with dysmenorrhea in a subject.

International Patent Application Publication No. WO 2014/121332 A1 discloses a cervical transfer catheter (10) for transferring a therapeutic substance to an endocervix or other internal mucosal surfaces of a recipient.

Tricyclic antidepressants, including amitriptyline (Elavil) and doxepin (Sinequan), have been reported as being useful in treating some patients with premenstrual anxiety or insomnia without frank depression (Davis, Clarissa Behr, and Michael Lee Davis. "The effects of premenstrual syndrome (PMS) on the female singer." Journal of Voice 7.4 (1993): 337-353).

CA2002182 C relates to the use of one or more serotonin-mediated neurotransmission enhancing drugs for the manufacture of a medicament, for treating disturbances of mood, disturbances of appetite, or both, associated with premenstrual syndrome in women.

Accordingly, there is a need for new treatment and management options for neuroinflammatory conditions. The present disclosure is directed to overcome and/or at least ameliorate one of more disadvantages of the prior art, and/or provide one or more advantages, or provide an alternative, as discussed herein.

### SUMMARY

The present invention is defined by the subject-matter set out in the appended claims. In particular, the present invention is directed to an agent that reduces activation of the innate immune system for use in a method of treating a female subject suffering from, or susceptible to, a neuroinflammatory condition, the method comprising intrauterine administration to the subject of an effective amount of the agent that reduces activation of the innate immune system and thereby treating the subject, wherein the agent is amitriptyline, wherein the neuroinflammatory condition is premenstrual syndrome (PMS) or premenstrual dysphoric disorder (PMDD), wherein the treatment improves or reduces an affective symptom and/or behavioural symptom in the subject, said symptom being selected from one or more mood swings, tearfulness, sensitivity to rejection, irritability or anger, marked depressed mood, hopelessness, self-deprecating thoughts, anxiety, tension difficulty concentrating and a sense of feeling overwhelmed.

In an embodiment, the agent reduces activation of spinal glial cells, the agent reduces activation of spinal glial astrocyte cells, and/or the agent reduces activation of circulating innate immune cells.

In an embodiment, the administration of amitriptyline to the subject comprises administration a dose of less than 100 µg/kg/day.

In an embodiment, the administration of the agent comprises long term continuous administration to the subject.

In an embodiment, the method further comprises administration of a sex hormone and/or an agent that modulates production and/or activity of a sex hormone.

In an embodiment, the intrauterine administration of the agent comprises release of the agent from an intrauterine device.

### BRIEF DESCRIPTION OF THE FIGURES

For a better understanding of the present disclosure, and to show more clearly how the present disclosure may be carried into effect according to one or more embodiments thereof, reference will be made, by way of example, to the accompanying figures.

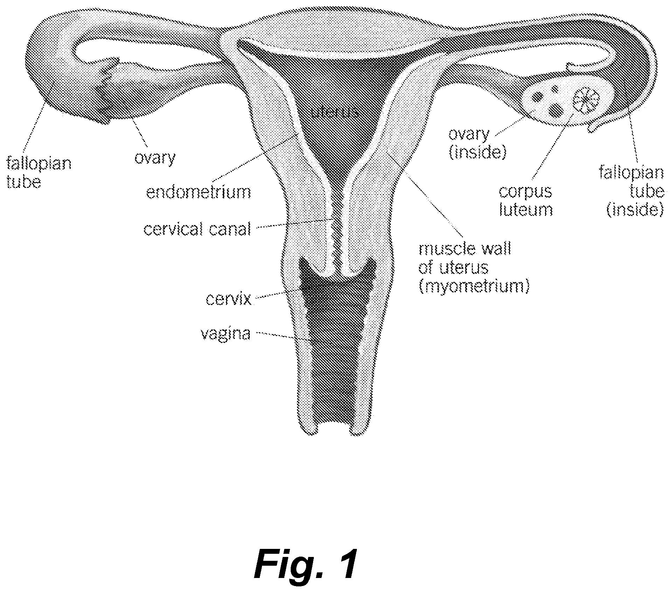

Figure 1 shows a representation of the gynaecological organs of the pelvis.

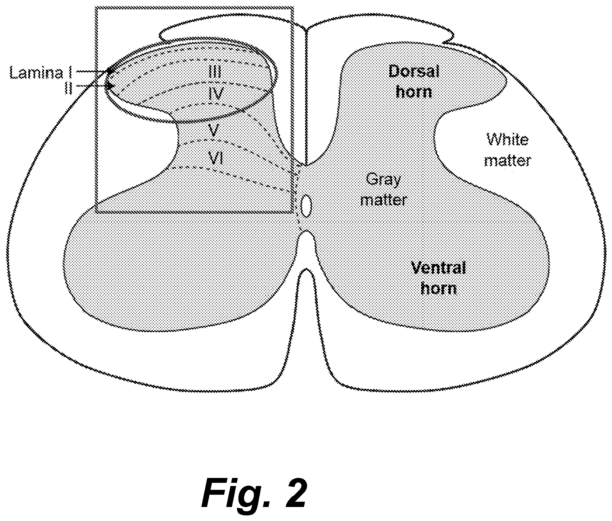

Figure 2 shows a schematic diagram of a transverse section of the mouse lumbar spinal cord. Major regions of white and gray matter, the dorsal and ventral horns, and locations of Rexed laminae I to VI within the dorsal horn are shown. The ellipsoid denotes the approximate position of ROIs used for measurements of glial immunoreactivity, and the box represents fields of view of images captured for analysis.

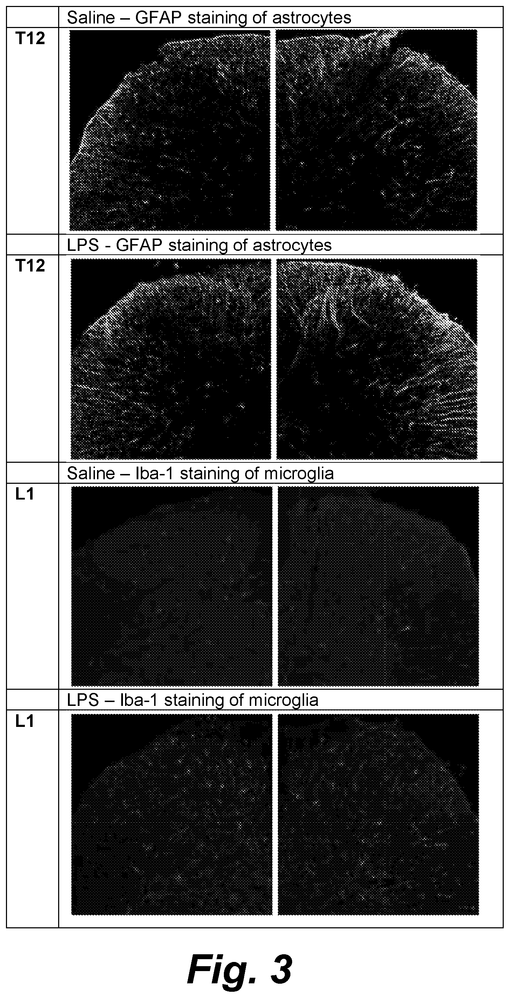

Figure 3 shows a comparison of dorsal horn staining of astrocytes and microglia following stimulation with either Saline or LPS. Intrauterine LPS induced a substantial increase in GFAP astrocyte staining throughout multiple levels of the spinal cord. Intrauterine LPS induced a localised increase in Iba1 microglial staining throughout select levels of the spinal cord.

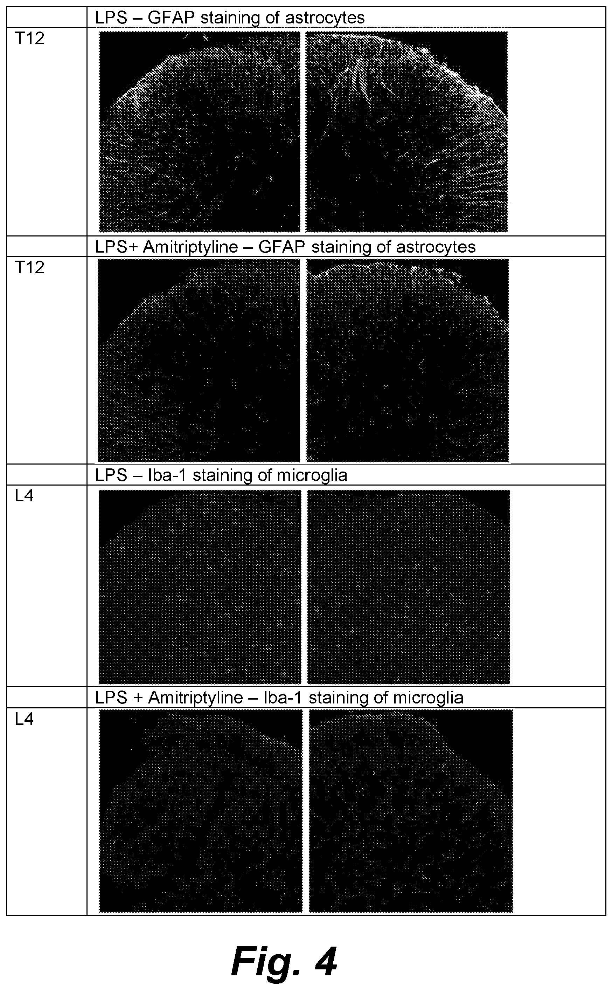

Figure 4 shows a comparison of dorsal horn staining of astrocytes and microglia following stimulation with either LPS, or LPS and amitriptyline. Amitriptyline treatment blocks the impact of intrauterine LPS at multiple levels of the spinal cord and appears to reduce astrocytic reactivity to below basal levels. Amitriptyline appears to reduce microglial reactivity to basal levels. Amitriptyline is an inhibitor of TLR2, TLR3, TLR4, TLR5, TLR8, TLR9, Dectin-1a, Dectin-1b, Mincle, NOD-1, NOD-2, RIG-1 and MDA-5.

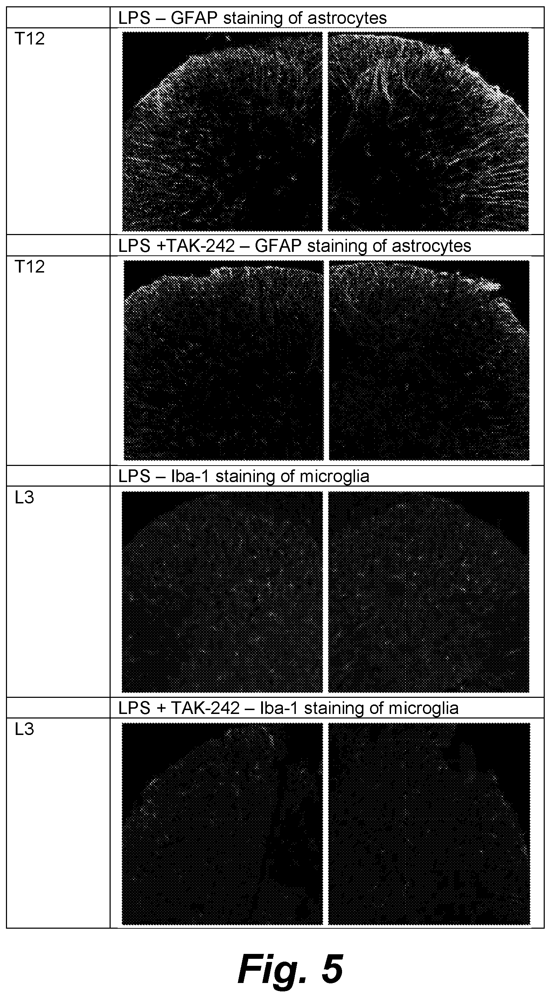

Figure 5 shows a comparison of dorsal horn staining of astrocytes and microglia following stimulation with either LPS, or LPS and TAK242. TAK242 treatment blocks the impact of intrauterine LPS at multiple levels of the spinal cord and appears to reduce astrocytic reactivity to below basal levels. TAK242 treatment appears to reduce microglia reactivity to basal levels. TAK242 is a pure inhibitor of TLR4.

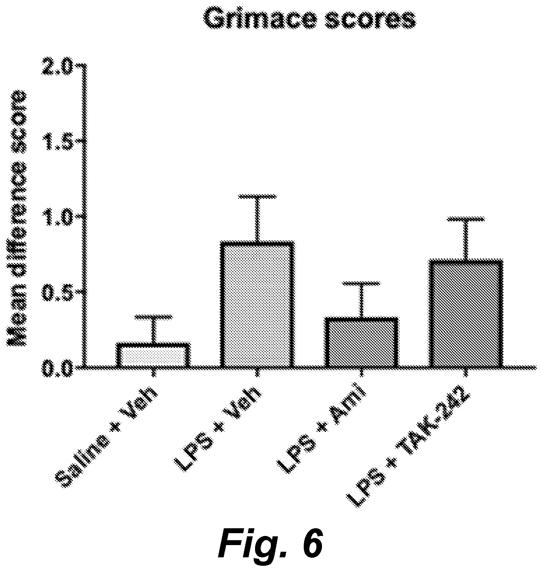

Figure 6 shows a comparison of grimace score in mice following intrauterine administration of saline only, LPS, LPS with amitriptyline and LPS with TAK242. LPS caused a significant increase in the presentation of Grimace behaviours in animals. Amitriptyline reduced these scores.

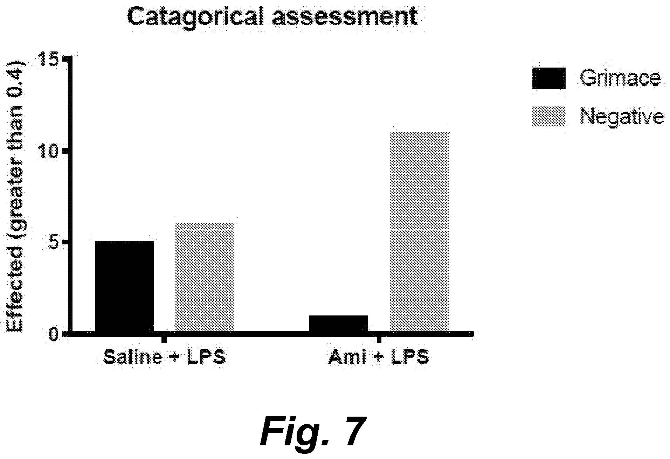

Figure 7 shows the proportion of mice displaying severe grimace following intrauterine administration of LPS only, or LPS with amitriptyline. Categorical assessment of grimace scores. Amitriptyline significantly reduced severe grimace behaviour. p= 0.043.

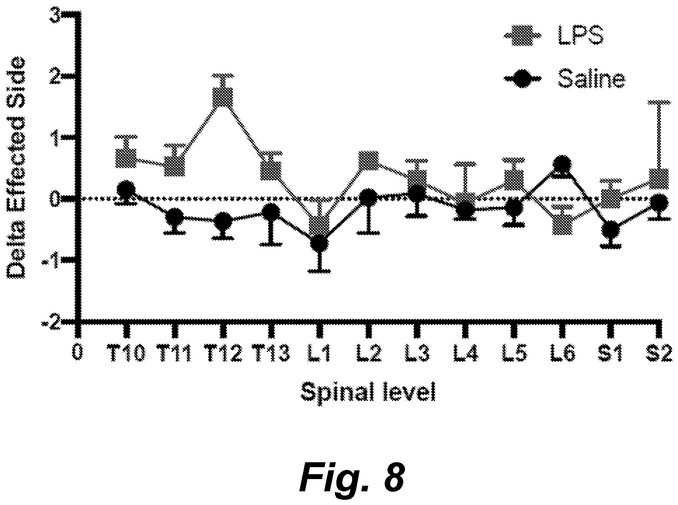

Figure 8 show a comparison of integrated density of activated astrocytes across multiple spinal cord levels following the intrauterine administration of saline only, or LPS. Intrauterine LPS induced activation of astrocytes at multiple levels of the spinal levels compared with saline, as measured by GFAP staining. 2 way ANOVA repeated measures demonstrated a main effect of treatment p = 0.0098.

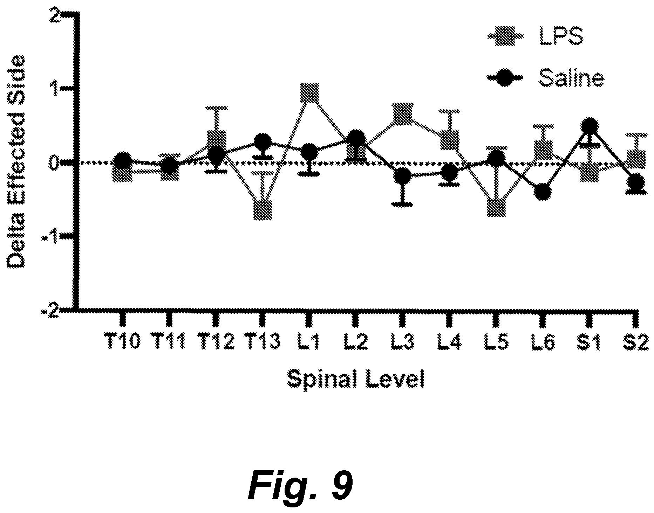

Figure 9 shows a comparison of integrated density of activated microglia across multiple spinal cord levels levels following the intrauterine administration of saline only, or LPS. Intrauterine LPS induced activation of microglia with inconsistent effect across levels. (Iba-1 staining). 2 way ANOVA repeated measures demonstrated a main effect of treatment p = 0.02.

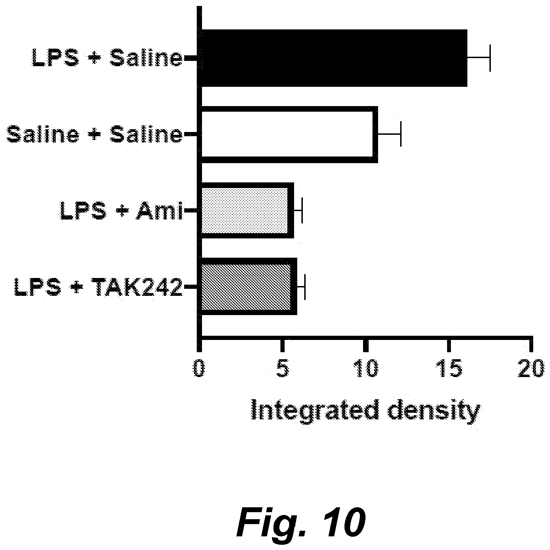

Figure 10 shows a comparison of integrated density of activated astrocytes within the dorsal horn following intrauterine administration of saline only, LPS, LPS and Amitriptyline, or LPS and TAK-242. Anova analysis of integrated density of GFAP staining at the level of T12 demonstrated a main effect of treatment p < 0.0001. Posthoc LPS effect p = 0.0176. Amitryptiline & TAK 242 both significantly reduce LPS effect, p = 0.0002.

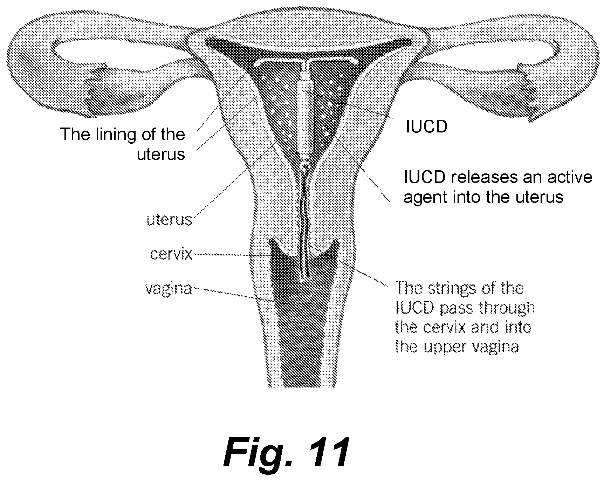

Figure 11 shows a representation of the pelvic organs with an intrauterine device according to certain exemplary embodiments for releasing an immune modulator within the uterine cavity. In the embodiment shown, the intrauterine device includes a drug reservoir attached to the shaft of the device, providing slow release of the agent to the endometrial cavity of the uterus.

### DETAILED DESCRIPTION

The references to methods of treatment by therapy of this description are to be interpreted as references to compounds, pharmaceutical compositions and medicaments of the present invention for use in those methods. Although, the present invention is defined by the subject-matter set out in the appended claims, the present disclosure relates, at least in part, to methods, compositions and devices for treating neuroinflammatory conditions in female subjects. The following disclosure provides teachings which go beyond the disclosure of the present invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the terms "embodiment", "invention", and "aspect" are not to be construed as necessarily referring to the claimed invention, unless the subject-matter in question falls within the scope of the claims. Methods of treatment *per se* are not a part of the claimed invention, but their disclosure herein relates to how an agent that reduces activation of the innate immune system, *i.e.,* amitriptyline, can be used in treating a female subject suffering from, or susceptible to, a neuroinflammatory condition.

The present disclosure is based, at least in part, on the recognition that neuroinflammatory conditions are associated with activation of glia within the nervous system and/or circulating immune cells. A treatment that reduces activation of glia within the nervous system or circulating immune cells is anticipated to reduce symptoms in a subject, and that uterine administration of agents that reduce activation of the innate immune system provides an alternative administration route for treating neuroinflammatory conditions outside of the uterus.

One or more embodiments of the present disclosure are directed to methods and products that have one or more combinations of the following advantages: new methods and/or products for treating neuroinflammatory conditions in women; methods of treating neuroinflammatory conditions using an uterine delivery route; methods of treating neuroinflammatory conditions using a delivery route that permits lower doses of a therapeutic agent than would be effective if taken orally or systemically; methods of treating a neuroinflammatory conditions using a delivery route that permits long term release of a therapeutic agent to be used; the use of therapeutic agents to treat neuroinflammatory conditions that have a safety profile established over many years; the incorporation of a new class of therapeutic agents previously unrecognised as being suitable for use in intrauterine devices; new methods for treating neuroinflammatory conditions in women wherein the treatment reduces development of opioid induced hyperalgesia and/or opioid tolerance; to address one or more problems, and/or to provide one or more advantages, or to provide a commercial alternative. Other advantages of certain embodiments of the present disclosure are also disclosed herein.

A representation of the female internal reproductive organs is shown in Figure 1, and a representation of the reproductive organs with an intrauterine device are shown in Figure 13.

Certain embodiments of the present disclosure provide a method of treating a female subject suffering from, or susceptible to, a neuroinflammatory condition.

In certain embodiments, the present disclosure provides a method of treating a female subject suffering from, or susceptible to, a neuroinflammatory condition, the method comprising intrauterine administration to the subject of an effective amount of an agent that reduces activation of the innate immune system and thereby treating the subject.

The term "condition" as used herein in relation to a medical condition refers to a disease, a disorder, an illness, a state, a precondition, or a physiologic, mental or psychological condition or disorder.

The term "treatment", and related terms such as "treating" and "treat" as used herein, refer to obtaining a desired pharmacologic and/or physiologic effect in terms of improving the condition of the subject, ameliorating, arresting, preventing, managing, suppressing, relieving and/or slowing the progression of one or more symptoms in the subject, a partial or complete stabilization of the subject, a regression of one or more symptoms, or a cure in the subject.

In certain embodiments of the present disclosure, the subject is suffering from a neuroinflammatory condition. In certain embodiments of the present disclosure, the subject is susceptible to a neuroinflammatory condition. In certain embodiments of the present disclosure, the subject is susceptible to progression of a neuroinflammatory condition.

In certain embodiments of the present disclosure, the neuroinflammatory condition is related to and/or exacerbated by the menstrual cycle.

In certain embodiments of the present disclosure, the method is used to reduce the intensity and/or the frequency of the condition. In certain embodiments of the present disclosure, the method is used to prevent or manage the condition. In certain embodiments of the present disclosure, the method is used to reduce the progression of the condition from a less severe state to a more severe state. In certain embodiments of the present disclosure, the method is used to provide an early intervention in a subject presenting with the condition.

In certain embodiments of the present disclosure, the subject is a human subject.

In certain embodiments of the present disclosure, the subject is an animal subject. Veterinary applications of the present disclosure are contemplated.

The term "an agent that reduces activation of the innate immune system" as used herein refers to an agent that directly or indirectly results in a reduction in the level of activation of the innate immune system, for example so as to cause a decrease in the level of activation, an inhibition of activation, a prevention of activation, a downregulation in the level of activation, a reduction in the ability to be stimulated, an alteration in the timing and/or location of activation, an alteration in downstream signalling, or otherwise provide some form of negative control over activation or combinations thereof. The innate immune system and its activation is described, for example, in Monie P. (2017) "The Innate Immune System: A Compositional and Functional Perspective", published by Elsevier. Methods for assessing whether an agent reduces activation of the innate immune system are known in the art. Agents for reducing activation of the innate immune system are known in the art, commercially available or can be identified by screening.

For example, the agent may (i) act to directly reduce activation, alter the level of expression of a target, alter localisation of a target, alter signalling, and/or alter timing of function, (ii) act to change the activity of a downstream signalling pathway associated with activation, (iii) act to alter the level and/or the activity of another molecule that regulates a target, such as by competitive/non-competitive binding, or by altering the synthesis, breakdown, and/or localisation of the other molecule. Other forms of action are contemplated, and combinations of forms of action are contemplated.

Examples of agents include a drug, a small molecule, a protein, a polypeptide, a lipid, a carbohydrate, a nucleic acid, an oligonucleotide, a ribozyme, a biologic, an aptamer, a cofactor, a ligand, a ligand mimetic, a receptor, a peptidomimetic, an enzyme, a kinase, a phosphatase, a cytokine, a growth factor, a metal ion, a chelate, an antisense nucleic acid, an inhibitor RNA, a microRNA, a siRNA, an antibody or an antigen binding part thereof, an antibody mimetic, a sex steroid, or combinations thereof. Other types of agents are contemplated. It will be appreciated than an agent as described herein also includes a prodrug of the agent, and/or a metabolite of the agent.

In certain embodiments of the present disclosure, the agent comprises a drug or small molecule, and/or a pro-drug or a metabolite thereof.

Methods for assessing that a subject is suffering from, or susceptible to, a neuroinflammatory condition are known in the art.

In certain embodiments of the present disclosure, the neuroinflammatory condition comprises an inflammatory pain condition.

In certain embodiments of the present disclosure, the neuroinflammatory condition comprises a non-uterine inflammatory pain condition. The term "a non-uterine inflammatory pain condition" as used herein refers to an inflammatory condition occurring outside of the uterus, and which may have an aetiology arising from either inside or outside of the uterus.

Examples of non-uterine inflammatory pain conditions include osteoarthritis, ankylosing spondylitis, gout (more common in males); chronic itch; chronic regional pain syndrome; post-herpetic neuralgia, inflammatory bowel disorders such as ulcerative colitis, Crohn's disease, irritable bowel syndrome, chronic opioid use (induced hyperalgesia); autoimmune conditions such as systemic lupus erythematosis, polyarteritis, myasthenia gravis, scleroderma, polymyositis, dermatomyositis, and antiphospholipid antibody syndrome; and fibromyalgia.

In certain embodiments of the present disclosure, the neuroinflammatory condition comprises an inflammatory condition of the central nervous system and/or the peripheral nervous system.

In this regard, there is increasing evidence that inflammation can travel in an antidromic direction and as such central nervous inflammation can induce peripheral nervous inflammation.

Examples of neuroinflammatory conditions of the central nervous system and/or the peripheral nervous system include demyelinating disorders, such as multiple sclerosis and acute disseminated encephalomyelitis; neurodegenerative disorders such as Alzheimer's disease, Prion disease and Parkinson's disease; brain injuries such as: stroke or brain trauma; complications associated with ageing, migraine, neuropsychiatric disorders such as depression, anxiety, premenstrual mood disorder, premenstrual dysphoric disorder, and schizophrenia; and Chronic Fatigue Syndrome.

In certain embodiments of the present disclosure, the neuroinflammatory condition is a mental or physical condition of the nervous system with increased prevalence in women, or which is related to and/or exacerbated by the menstrual cycle.

In certain embodiments of the present disclosure, the neuroinflammatory condition comprises Premenstrual Syndrome (PMS) or Premenstrual dysphoric disorder (PMDD).

In certain embodiments of the present disclosure, the method improves or reduces an affective symptom and/or a behavioural symptom in the subject. In certain embodiments of the present disclosure, the symptoms comprises one or more mood swings, tearfulness, sensitivity to rejection, irritability or anger often characterized by increased interpersonal conflicts, marked depressed mood, hopelessness, self-deprecating thoughts, anxiety, tension or feeling on edge, difficulty concentrating and a sense of feeling overwhelmed or out of control.

In certain embodiments of the present disclosure, the neuroinflammatory condition is migraine.

In certain embodiments of the present disclosure, the method reduces one or more of the severity of migraine, reduces the frequency of episodes of migraine, reduces the length of the migraine, reduces one or more pain symptoms associated with migraine, and/or reduces the probability of progression to a migraine.

In certain embodiments of the present disclosure, the method reduces development of opioid induced hyperalgesia and/or opioid tolerance, which are considered to have, at least in part, a neuroinflammatory component. Methods for assessing opioid induced hyperalgesia and/or opioid tolerance are known in the art. In certain embodiments of the present disclosure, the method of treatment provides a form of treatment that reduces development of opioid induced hyperalgesia and/or opioid tolerance. In certain embodiments of the present disclosure, the method of treatment reduces development of opioid induced hyperalgesia and/or opioid tolerance in the subject.

In certain embodiments of the present disclosure, the agent reduces activation of spinal glial cells. Methods for assessing whether an agent reduces activation of spinal glial cells are known in the art and described herein.

In certain embodiments, of the present disclosure the agent that reduces spinal glial activation comprises an agent that reduces activation of spinal astrocytes. In certain embodiments of the present disclosure, the agent that reduces spinal glial activation comprises an agent that reduces activation of spinal microglia. In certain embodiments of the present disclosure, the agent that reduces spinal glial activation comprises an agent that reduces activation of spinal astrocytes and spinal microglia.

In certain embodiments of the present disclosure, the agent reduces activation of circulating innate immune cells. Methods for assessing whether an agent reduces activation of circulating innate immune cells are known in the art and described herein. Agents for reducing activation of circulating innate immune cells are known in the art, commercially available or can be identified by screening.

In certain embodiments of the present disclosure, the agent is a direct or indirect inhibitor of a receptor. In certain embodiments of the present disclosure, the inhibitor is a selective inhibitor. In certain embodiments of the present disclosure, the inhibitor is a non-selective inhibitor.

In certain embodiments of the present disclosure, the agent is a direct or indirect receptor antagonist. In certain embodiments of the present disclosure, the antagonist is a selective antagonist. In certain embodiments of the present disclosure, the antagonist is a non-selective antagonist.

In certain embodiments of the present disclosure, the agent reduces activation of a pattern recognition receptor. Pattern recognition receptors include Toll-like receptors (TLRs), C-type lectin receptors, NOD-like receptors, Retinoic acid-inducible gene-1-like receptors (RIG-1-like receptors), and Melanoma-differentiation-associated gene 5 receptors (MDA-5)

In certain embodiments of the present disclosure, the agent comprises an inhibitor of a pattern recognition receptor. Pattern recognition receptors are described, for example, Deswaerte et al. (2017) Mol. Immunology 86: 3-9. Agents for reducing activation of pattern recognition receptors are known in the art, commercially available or can be identified by screening. Inhibitors and antagonists of pattern recognition receptors are described, for example, in Mullen et al. (2015) Arthritis Res Ther. 17(1): 122.

Examples of pattern recognition receptor antagonists include NI-0101 (TLR4 target; antibody), Chaperonin 10 (TLR4 antagonist; protein), VTX-763 (TLR8 target; small molecule), CRID3 (NLRP3 target; small molecule), OPN-305 (TLR2 target; antibody). IMO-3100 (TLR7/TLR9 target; DNA based small molecule), DV1179 (TLR7/TLR9 target; small molecule), and CPG52364 (TLR7/TLR9 target; small molecule).

In certain embodiments of the present disclosure, the agent comprises an inhibitor or an antagonist of a Toll-like Receptor (TLR). Functions of Toll-like receptors and their downstream signalling events are known in the art, for example as described in Barton GM, Kagan JC (2009) Nat. Rev. Immunol. 9(8), 535-42; Blasius AL, Beutler B (2010) Immunity 32(3), 305-15; Kawai T, Akira S (2010) Nat. Immunol. 11(5), 373-84; Lester SN, Li K (2014) J. Mol. Biol. 426(6), 1246-64; Li X, Jiang S, Tapping RI (2010) Cytokine 49(1), 1-9; McGettrick AF, O'Neill LA (2010) Curr. Opin. Immunol. 22(1), 20-7; Miggin SM, O'Neill LA (2006) J. Leukoc. Biol. 80(2), 220-6; Pasare C, Medzhitov R (2005) Adv. Exp. Med. Biol. 560, 11-8; and Reuven EM, Fink A, Shai Y (2014) Biochim. Biophys. Acta 1838(6), 1586-93. Methods for identifying agents that inhibit or antagonise Toll-like receptors are known in the art, and described herein. Agents that inhibit or antagonise a Toll-like receptor are known in the art, commercially available, or can be identified by screening.

In certain embodiments of the present disclosure, the agent comprises an inhibitor or an antagonist of a C-type lectin receptor (CLR). C-Type lectin receptors are described, for example, in Del Fresno et al. (2018) Front Immunol. 9: 804. Methods for identifying agents that inhibit or antagonise C-type lectin receptors are known in the art, and described herein. Agents that inhibit or antagonise C-type lectin receptors are known in the art, commercially available or can be identified by screening.

In certain embodiments of the present disclosure, the agent comprises an inhibitor or an antagonist of a NOD-like receptor (NDR). NOD-like receptors are described, for example, in Platnich and Muruve (2019) Arch Biochem Biophys. 670:4-14 and in Feerick and McKernan (2017) Immunology 150(3): 237-247. Methods for identifying agents that inhibit or antagonise NOD-like receptors are known in the art, and described herein. NOD-like receptors are described, for example, in Feerick and McKernan (2017) Immunology 150(3): 237-247. Agents that inhibit or antagonise NOD-like receptors are known in the art, commercially available or can be identified by screening.

Examples of antagonist/inhibitors of NOD-like receptors include MCC950 (selective NLRP3 inhibitor), CY-09 (selective and direct NLRP3 inhibitor), NOD-IN-1 (inhibitor of NOD1 and NOD2), Nodinitib-1 (NOD1 inhibitor), and YQ128 selective NLRP3 inhibitor).

In certain embodiments of the present disclosure, the agent comprises an inhibitor of a Toll-like receptor (TLR), a C-type lectin receptor (CLR), a NOD-like receptor (NDR), a RIG-1-like receptor (RIG-1 receptor) and/or an MDA-5 receptor.

In certain embodiments of the present disclosure, the agent comprises an inhibitor of one of more TLR4 (GeneCard GCID: GC09P117704), TLR3 (GeneCard GCID: GC04P186059), TLR2 (GeneCard GCID: GC04P153684), TLR5 (GeneCard GCID:GC01M223109), TLR8 (GeneCard GCID: GC0XP01292), TLR9 (GeneCard GCID: GC03M052222), Dectin-1a (GeneCard GCID: GC12M013368), Dectin 1-b (GeneCard GCID: GC12M013368) Mincle (GeneCard GCID:GC12M008535), NOD1 (GeneCard GCID:GC12M008535), and NOD2 (GeneCard GCID: GC16P050693), RIG-1 (GeneCard GCID:GC09M032455) or MDA-5 (GeneCard GCID:GC02M162267.

In certain embodiments of the present disclosure, the agent that reduces activation of the innate immune system comprises a TLR4 inhibitor. In certain embodiments of the present disclosure, the TLR4 inhibitor is a selective inhibitor. In certain embodiments of the present disclosure, the TLR4 inhibitor is a non-selective inhibitor.

In certain embodiments of the present disclosure, the agent that reduces activation of the innate immune system comprises a TLR4 antagonist. In certain embodiments of the present disclosure, the TLR4 antagonist is a selective antagonist. In certain embodiments of the present disclosure, the TLR4 antagonist is a non-selective antagonist.

TLR4 inhibitors and antagonists are known, and are commercially available or may be produced by a method known in the art. Methods for determining whether an agent is a TLR4 inhibitor or antagonist are known in the art, for example as described in Coats SR. et al. (2005). J Immunol. 175(7):4490-8*.*

In certain embodiments of the present disclosure, the TLR4 inhibitor or antagonist comprises a small molecule. In certain embodiments of the present disclosure, the TLR4 inhibitor or antagonist comprises an antibody and/or an antigen binding part thereof. In certain embodiments of the present disclosure, the TLR4 inhibitor or antagonist comprises a nucleic acid.

Examples of TLR4 inhibitors or antagonists include one or more of Eritoran, Amitriptyline (for example available from Mylan Pharmaceuticals Inc, USA), Nortriptyline (for example available from Centaur Pharmaceuticals), Cyclobenzaprine (for example available from Jubilant Life Sciences), Ketotifen (for example available from Sifavitor), Imipramine (for example available from H. Lundbeck AS), Mianserin (for example available from Albany Molecular Research Inc.), Ibudilast (for example available from Sanyo Chemical Laboratory Ltd), Pinocembrin, (+)Naltrexone,(-) Naltrexone, (+) Naloxone, (-) Naloxone, minocycline (for example available from Albany Molecular Research Inc.), LPS-RS, Propentofylline (for example Labratorio Chimico Internazionale Spa) and (+)-naloxone, 1J, TAK-242, Desipramine (for example available from H. Lundbeck AS), Carbamazepine (for example available from SAFC, Sigma-Aldrich Corporation) Oxcarbazepine (for example available from Albany Molecular Research Inc.), Rimcazole, Mesoridazine (for example available from Sumika Fine Chemicals Co Ltd), Tacrine (for example available from Nordic Syhtnesis AB), Orphenadrine (for example available from Kores India Limited), Diphenhydramine (for example available from Cadila Pharmaceuticals Limited, Duloxetine (for example available from BOC Sciences), Venlafaxine (for example available from Macleods Pharmaceuticals Limited), Chlorpromazine (for example available from Egis Pharmaceuticals PLC), Fluoxetine (for example available from PRONOVA BIOPHARMA NORGE AS), curcumin, an effective statin, an effective cannabinoid, and compounds Lipid A mimetic, SPA4, STM28, xanthohumal, JTT-705, auranofin, sulforaphane, cinnamaldehyde, taxanes, 6-shogaol, soliquiritigenin, OSL07, glycyrrhizin, isoliquiritigenin, caffeic acid phenethyl ester, IAXO-101, T5342126, KRGISPGGGSDAQGEV, morphine, NCI126224, paclitaxel, heme, chitohexaose, compounds 12 to 18, 22, and 29 to 33 as described in Wang et al (2013) Chem Soc Rev. 42(12): 4859-4866.

In certain embodiments of the present disclosure, the TLR4 inhibitor comprises amitriptyline and/or nortriptyline.

In certain embodiments of the present disclosure, the TLR4 inhibitor comprises TAK242.

In certain embodiments of the present disclosure, the TLR4 antagonist comprises one or more of amitriptyline, nortriptyline and TAK-242.

In certain embodiments of the present disclosure, the agent that reduces activation of the innate immune system comprises a TLR2 inhibitor. In certain embodiments of the present disclosure, the TLR2 inhibitor is a selective inhibitor. In certain embodiments of the present disclosure, the TLR2 inhibitor is a non-selective inhibitor.

In certain embodiments of the present disclosure, the agent that reduces activation of the innate immune system comprises a TLR2 antagonist. In certain embodiments of the present disclosure, the TLR2 antagonist is a selective antagonist. In certain embodiments of the present disclosure, the TLR2 antagonist is a non-selective antagonist.

TLR2 inhibitors and antagonists are known, and are commercially available or may be produced by a method known in the art. Examples of TLR2 inhibitors or antagonists include one or more of tricyclics including amitriptyline (for example available from Mylan Pharmaceuticals Inc, USA), and agents such as CU-CPT22 (for example available from Calbiochem), Sparstolonin B (available from Sigma Aldrich), and sulphoglycolipids, and compounds 1 to 5 as described in Wang et al (2013) Chem Soc Rev. 42(12): 4859-4866. Methods for determining whether an agent is a TLR2 inhibitor or antagonist are known in the art, for example as described in Cheng, K., et al. 2012. Angew. Chem. Int. Ed. 51, 12246.

In certain embodiments of the present disclosure, the TLR2 inhibitor or antagonist comprises a small molecule. In certain embodiments of the present disclosure, the TLR2 inhibitor or antagonist comprises an antibody and/or an antigen binding part thereof. In certain embodiments of the present disclosure, the TLR2 inhibitor or antagonist comprises a nucleic acid.

In certain embodiments of the present disclosure, the TLR2 antagonist comprises amitriptyline.

In certain embodiments of the present disclosure, the agent comprises a TLR4 antagonist and/or a TLR2 antagonist.

In certain embodiments of the present disclosure, the inhibitor or antagonist is both a TLR2 inhibitor or antagonist and a TLR4 inhibitor or antagonist. Amitriptyline is an example of a drug that is a TLR4 and TLR2 antagonist.

In certain embodiments of the present disclosure, the agent that reduces activation of the innate immune system comprises one or more of a TLR4 inhibitor, a TLR2 inhibitor, minocycline, fluorocitrate, and propentofylline.

Examples of TLR3 antagonists include CNTO4685, CNTO5429, CNTO4685, hydroxychloroquine, imidazoquinolines, and propidium iodide. Examples of TLR8 inhibitors include hydroxychloroquine sulfate, CU-CPT-8, ODN 2088, and CU-CPTRm. Examples of TLR9 antagonists include ODN 2088, ODN 4084-F, COV08-0064, imidazoquinolines, propidium iodide, IMO-3100, IRS-954, E6446, and DV1179.

The term "intrauterine administration" as used herein refers to administration of an agent by way of the uterus, the cervix, and/or the cervical canal (see for example Figure 1).

The term "effective amount" as used herein refers to that amount of an agent that is sufficient to effect treatment, when administered to a subject. The effective amount will vary depending upon a number of factors, including for example the specific activity of the agent being used, the severity of the condition, the subject, the age, physical condition, existence of other disease states, nutritional status of the subject and genetic background of the subject.

In this regard, it has been found that the effective dose for agents that reduce activation of the innate immune system by intrauterine administration is markedly lower than would have been anticipated from studies using other administration routes, such as oral administration. For example, it was found in the present studies that amitriptyline is effective in a mouse model at reducing spinal glial activation at a dose of 27 µg/kg, and that TAK242 is effective at a concentration of 36 µg/kg.

The lower effective dose required is desirable, as some agents carry significant adverse effects at higher doses.

For example, amitriptyline is a known drug with extensive use over decades and a known safety profile, but which still has a number of adverse effects: When taken orally, amitriptyline has adverse effects such as drowsiness, dry mouth, blurred vision, pupil dilation, constipation, weight and urinary retention. Use of a lower dose obviates, or at least reduces, these side effects.

In certain embodiments of the present disclosure, the agent is administered to the subject in an amount ranging from one of the following selected ranges: 1 µg/kg to 10 mg/kg; 1 µg/kg to 1 mg/kg; 1 µg/kg to 100 µg/kg; 1 µg/kg to 10µg/kg; 10 µg/kg to 10 mg/kg; 10 µg/kg to 1 mg/kg; 10 µg/kg to 100 µg/kg; 100 µg/kg to 10 mg/kg; 100 µg/kg to 1 mg/kg; or 1 mg/kg to 10 mg/kg. Other ranges are contemplated.

In certain embodiments of the present disclosure, the agent is administered to the subject in an amount ranging from one of the following selected ranges: 0.01 µg/kg/day to 10 mg/kg/day, 0.01 µg/kg/day to 1 mg/kg/day, 0.01 µg/kg/day to 100 µg/kg/day, 0.01 µg/kg/day to 10 µg/kg/day 0.01 µg/kg/day to 1 µg/kg/day, 0.01 µg/kg/day to 0.1 µg/kg/day, 0.1 µg/kg/day to 10 mg/kg/day, 0.1 µg/kg/day to 1 mg/kg/day, 0.1 µg/kg/day to 100 µg/kg/day, 0.1 µg/kg/day to 10 µg/kg/day 0.1 µg/kg/day to 1 µg/kg/day, 1 µg/kg/day to 10 mg/kg/day; 1 µg/kg/day to 1 mg/kg/day; 1 µg/kg/day to 100 µg/kg/day; 1 µg/kg/day to 10µg/kg/day; 10 µg/kg/day to 10 mg/kg/day; 10 µg/kg/day to 1 mg/kg/day; 10 µg/kg/day to 100 µg/kg/day; 100 µg/kg/day to 10 mg/kg/day; 100 µg/kg/day to 1 mg/kg/day; or 1 mg/kg/day to 10 mg/kg/day. Other ranges are contemplated.

In certain embodiments of the present disclosure, the method comprises administration to the subject of a dose of the agent of less than 100 µg/kg. In certain embodiments of the present disclosure, the method comprises administration to the subject of a dose of the agent of less than 70 µg/kg. In certain embodiments of the present disclosure, the method comprises administration to the subject of a dose of the agent of less than 50 µg/kg. In certain embodiments of the present disclosure, the method comprises administration to the subject of a dose of the agent of less than 25 µg/kg. In certain embodiments of the present disclosure, the method comprises administration to the subject of a dose of the agent of less than 10 µg/kg.

In certain embodiments of the present disclosure, the method comprises administration to the subject of a dose of the agent of less than 100 µg/kg/day. In certain embodiments of the present disclosure, the method comprises administration to the subject of a dose of the agent of less than 70 µg/kg/day. In certain embodiments of the present disclosure, the method comprises administration to the subject of a dose of the agent of less than 50 µg/kg/day. In certain embodiments of the present disclosure, the method comprises administration to the subject of a dose of the agent of less than 25 µg/kg/day. In certain embodiments of the present disclosure, the method comprises administration to the subject of a dose of the agent of less than 10 µg/kg/day.

In certain embodiments of the present disclosure, the agent is a TLR4 and/or TLR2 inhibitor or antagonist, and the agent is administered in an amount from 10 µg/kg to 100 µg/kg, or 10 µg/kg to 50 µg/kg. In certain embodiments of the present disclosure, the agent is a TLR4 inhibitor or antagonist, and the agent is administered in an amount from 10 µg/kg/day to 100 µg/kg/day or 10 µg/kg/day to 50 µg/kg/day. Other ranges are contemplated.

In certain embodiments of the present disclosure, the method comprises administration to the subject of a TLR4 or TLR2 inhibitor or antagonist at a dose of less than 100 µg/kg, less than 70 µg/kg, less than 50 µg/kg, less than 25 µg/kg, or less than 10 µg/kg.

In certain embodiments of the present disclosure, the method comprises administration to the subject of a TLR4 and/or TLR2 inhibitor or antagonist at a dose of less than 100 µg/kg/day, less than 70 µg/kg/day, less than 50 µg/kg/day, less than 25 µg/kg/day, or less than 10 µg/kg/day.

In certain embodiments of the present disclosure, the present disclosure provides a low dose method of treating a female subject suffering from, or susceptible to, a neuroinflammatory condition, by local targeting of the immune cells within the uterus.

In certain embodiments of the present disclosure, the present disclosure provides a low dose method of treating a female subject suffering from, or susceptible to, a neuroinflammatory condition, the method comprising intrauterine administration to the subject of an effective amount of an agent that reduces activation of the innate immune system and thereby treating the subject.

In certain embodiments of the present disclosure, the present disclosure provides a method of treating a female subject suffering from, or susceptible to, a neuroinflammatory condition, the method comprising intrauterine administration to the subject of an effective amount of an agent that reduces activation of the innate immune system that is lower than would be required when administered by a systemic route.

The agent may be administered to the subject in a suitable form. In this regard, the term "administering" as used herein includes administering the agent, or administering a prodrug, or a derivative that will form an effective amount of the agent at the site of action. The terms include various types of administration forms such as liquid compositions, semi-solid compositions, suppositories, gels, solids, tablets, capsules, creams, solutions, pastes, ointments, implants or by way of a release from a device. Other administration forms are contemplated.

Methods for intrauterine administration of agents generally are as described, for example, in Sahoo et al (2013) American Journal of Advanced Drug Delivery: ISSN-2321-547X, Bhowmik et al (2010) Annals of Biological Research 1(1): 70-75, and Widermeesch D. (2010) Hand. Exp Pharmacol. 197: 268-298.

The agent may be administered alone or may be delivered in a mixture with other therapeutic agents and/or agents that, for example, enhance, stabilise or maintain the activity of the agent.

In this regard, the subject may be treated or given another drug or treatment modality in conjunction with the agent as described herein. It will be appreciated that the administration of another drug need not be delivered to the uterus, but may also be by other administration routes such as orally, intravenously, by injection, peritoneally, by implant, or by way of suppository. Examples include levonorgestrel, or an anti-inflammatory agent such as naprosyn or diclofenac.

Combination therapy with other agents can be sequential therapy where the subject is treated first with one and then the other, or the two or more treatment modalities are given simultaneously. For example, two or more therapeutic agents can be co-formulated into a single dosage form or "combined dosage unit", or formulated separately and subsequently combined into a combined dosage unit.

In certain embodiments of the present disclosure, the administering to the subject comprises continuous administering to the subject of the agent.

In certain embodiments of the present disclosure, the administering to the subject comprises a dose of the agent administered on a regular basis, such as daily, twice daily, weekly, monthly, annually, or multi-annually.

In certain embodiments of the present disclosure, the administering to the subject comprises escalating doses of the agent, decreasing doses of the agent, and/or repeated doses.

In certain embodiments of the present disclosure, the administering to the subject comprises long-term administration of the agent to the subject. In certain embodiments of the present disclosure, the administering to the subject comprises long-term continuous administration of the agent to the subject.

In certain embodiments of the present disclosure, the agent is administered as an immediate release formulation. The term "immediate release formulation" as used herein is a formulation designed to quickly release an agent in the body over a shortened period of time. Immediate release formulations are known in the art.

In certain embodiments of the present disclosure, the agent is administered as a slow release/sustained release formulation. The term "sustained release formulation" as used herein is a formulation designed to slowly release an agent in the body over an extended period of time. Sustained release formulations are known in the art.

In certain embodiments of the present disclosure, the agent is administered by way of release from a composition. In certain embodiments of the present disclosure, the agent that is administered by way of release from a substrate. In certain embodiments of the present disclosure, the agent is administered by way of release through a membrane.

In certain embodiments of the present disclosure, the agent that reduces activation of the innate immune system is administered by way of release from a device.

In certain embodiments of the present disclosure, the agent that reduces activation of the innate immune system is administered to the subject in a composition suitable for intrauterine administration, as described herein.

In certain embodiments of the present disclosure, the agent that reduces activation of the innate immune system is administered to the subject via a liquid composition, a semi-solid composition, a gel, a solid, a suppository, a tablet, a capsule, a cream, a solution, a paste, or an ointment. Other compositional forms are contemplated.

In certain embodiments of the present disclosure, the agent that reduces activation of the innate immune system is administered to the subject via a device. In certain embodiments of the present disclosure, the administration comprises release from a device.

Devices for intrauterine administration of agents are known in the art. Examples of devices include, for example, devices sold under the brand name "Mirena". Devices are described, for example, in Bandyopadhyay A.K. (2008), Novel drug delivery systems, 1st edition, Everest publishing house, p. 215-220 and Bao et al. (2018) Int. J. Pharm. 550(1-2): 447-453.

Substrates for use in devices for delivery of active agents are known in the art, and include for example, copolymers of di-methylsiloxanes and methylvinylsiloxanes, ethylene/vinyl acetate copolymers (EVA), polyethylene, polypropylene, ethylene/propylene copolymers, acrylic acid polymers, ethylene/ethyl acrylate copolymers, polytetrafluoroethylene (PTFE), polyurethanes, thermoplastic polyurethanes and polyurethane elastomers, polybutadiene, polyisoprene, poly(methacrylate), polymethyl methacrylate, styrene-butadiene-styrene block copolymers, poly(hydroxyethyl-methacrylate) (pHEMA), polyvinyl chloride, polyvinyl acetate, polyethers, polyacrylo-nitriles, polyethylene glycols, polymethylpentene, polybutadiene, polyhydroxy alkanoates, poly(lactic acid), poly(glycolic acid), polyanhydrides, polyorthoesters, hydrophilic polymers such as the hydrophilic hydrogels, cross-linked polyvinyl alcohol, neoprene rubber, butyl rubber, hydroxyl-terminated organopolysiloxanes, and copolymers of the aforementioned. Delivery of agents may also be accomplished by incorporation of the agent into the structural framework of a device. Methods for incorporating active agents into a substrate for intrauterine release are known in the art.

In certain embodiments of the present disclosure, the method further provides administering a sex hormone and/or an agent that modulates production and/or activity of a sex hormone directly or indirectly, such as a GnRH agonist or antagonist. The administering may be intrauterine and/or may utilise another route of administration, such as oral administration or a non-oral administration.

Sex hormones and/or an agents that modulate production and/or activity of a sex hormone may be natural or synthetic agents, and includes for example steroids such as gonadocorticoids, agents that interact with estrogen, progesterone or androgen receptors such as selective estrogen receptor modulators (SERM), selective progesterone receptor modulators (SPRM), selective androgen receptor modulators (SARM), and/or other agents that have the ability to modulate activity associated with a sex hormone.

In certain embodiments of the present disclosure, the method further comprises local pelvic administration to the subject of a sex hormone and/or an agent that modulates production and/or activity of a sex hormone, such as an estrogen, a progestogen, an androgen, a SERM, a SPRM and/or a SARM. A suitable dose and treatment regime may be selected. In certain embodiments of the present disclosure, the method further comprises intrauterine administration of a sex hormone and/or an agent that modulates production and/or activity of a sex hormone.

In certain embodiments of the present disclosure, the sex hormone comprises an estrogen and/or a progestogen. In certain embodiments of the present disclosure, the sex hormone comprises an androgen.

Examples of estrogen compounds include steroidal and non-steroidal estrogen compounds. Examples of progestogen compounds include one or more of the following compounds: progesterone and its derivatives, dienogest, cyproterone acetate, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17α-ethinyltestosterone and derivatives thereof, 17α-ethinyl-19-nor-testosterone and derivatives thereof, ethynodiol diacetate, dydrogesterone, norethynodrel, allylestrenol, medrogestone, norgestrienone, ethisterone and dl-norgestrel.

In certain embodiments of the present disclosure, the progestogen comprises one or more of levonorgestrel, dienogest, or a GnRH agonist or antagonist.

Examples of androgen compounds include steroidal and non-steroidal androgenic compounds.

In certain embodiments of the present disclosure, the sex hormone and/or the agent that modulates production and/or activity of a sex hormone is administered to the subject via the same administration route as the agent that reduces activation of the innate immune system.

In certain embodiments of the present disclosure, the sex hormone and/or the agent that modulates production and/or activity of a sex hormone is administered by local pelvic administration. In certain embodiments of the present disclosure, the sex hormone and/or the agent that modulates production and/or activity of a sex hormone is administered by way of a composition suitable for local pelvic administration. In certain embodiments of the present disclosure, the sex hormone and/or the agent that modulates production and/or activity of a sex hormone is administered by way of an intrauterine composition and/or a vaginal composition. Compositions are described herein.

In certain embodiments of the present disclosure, the sex hormone and/or the agent that modulates production and/or activity of a sex hormone is administered to the subject by way of release from a pelvic device. In certain embodiments of the present disclosure, the sex hormone and/or the agent that modulates production and/or activity of a sex hormone is administered to the subject by way of release from an intrauterine device and/or a vaginal device. Devices are described herein.

**In** certain embodiments of the present disclosure, the device provides long term release of a sex hormone and/or the agent that modulates production and/or activity of a sex hormone. In certain embodiments of the present disclosure, the device provides long term continuous release of a sex hormone and/or the agent that modulates production and/or activity of a sex hormone.

Certain embodiments of the present disclosure provide an intrauterine composition for treating a female subject suffering from, or susceptible to, a neuroinflammatory condition.

In certain embodiments, the present disclosure provides a composition for intrauterine administration for treating a female subject suffering from, or susceptible to, a neuroinflammatory condition, the composition comprising an agent that reduces activation of the innate immune system.

Neuroinflammatory conditions are described herein. In certain embodiments of the present disclosure, the neuroinflammatory condition comprises an inflammatory pain condition. In certain embodiments of the present disclosure, the neuroinflammatory condition comprises an inflammatory condition of the central nervous system and/or the peripheral nervous system.

Compositions for intrauterine administration are described herein. Agents that reduce activation of the innate immune system are described herein.

In certain embodiments of the present disclosure, the agent reduces activation of a pattern recognition receptor. Pattern recognition receptors and agents that reduce activation of pattern recognition receptors are described herein.

In certain embodiments of the present disclosure, the agent reduces activation of spinal glial cells. Agents that reduce activation of spinal glial cells are described herein.

In certain embodiments of the present disclosure, the agent reduces activation of circulating innate immune cells. Agents that reduce activation of circulating innate immune cells are described herein.

In certain embodiments of the present disclosure, the agent comprises an inhibitor of a Toll-like receptor (TLR), a C-type lectin receptor (CLR), a NOD-like receptor (NDR), a RIG-1 receptor and/or a MDA-5 receptor. Such agents are described herein.

In certain embodiments of the present disclosure, the agent comprises an inhibitor of one of more TLR2, TLR3, TLR4, TLR5, TLR8, TLR9, Dectin-1a, Dectin-1b, Mincle, NOD-1, NOD-2, RIG-1 and MDA-5. Such agents are described herein.

In certain embodiments of the present disclosure, the agent comprises a TLR4 antagonist and/or a TLR2 antagonist. TLR4 and TLR2 antagonists are described herein.

In certain embodiments of the present disclosure, the agent comprises one or more of amitriptyline, nortriptyline and TAK-242.

In certain embodiments of the present disclosure, the composition comprises a liquid composition, a semi-solid composition, a suppository, a gel, a solid, a tablet, a capsule, a cream, a solution, a paste, or an ointment. In certain embodiments of the present disclosure, the composition comprises a substrate with a releasable agent that reduces activation of the innate immune system.

Compositions for intrauterine administration are known in the art, for example as described in Sahoo et al (2013) American Journal of Advanced Drug Delivery: ISSN-2321-547X.

Additional numerous various excipients, dosage forms, dispersing agents and the like that are suitable for use in connection with administration and/or the formulation into medicaments or compositions are known and described in, for example, Remington's Pharmaceutical Sciences, 17th ed., Mack Publishing Company, Easton, Pa., 1985, which is incorporated herein by reference in its entirety. Methods for formulating compositions are known in the art.

A suitable dosage of the agent that reduces activation of the innate immune system in the composition may be selected. In certain embodiments of the present disclosure, the composition comprises the agent in an amount ranging from 10 µg to 500 mg, 10 µg to 100 mg, 10 µg to 10 mg, 10 µg to 1 mg, 10 µg to 100 µg, 100 µg to 500 mg, 100 µg to 100 mg; 100 µg to 10 mg; 100 µg to 1 mg, 1 mg to 500 mg 1 mg to 100 mg, or 1 mg to 10 mg. Other ranges are contemplated. Other amounts are contemplated.

In certain embodiments of the present disclosure, the composition comprises the agent in an amount to provide a dose ranging from one of the following selected ranges: 0.1 µg/kg to 10 mg/kg; 0.1 µg/kg to 1 mg/kg; 0.1 µg/kg to 100 µg/kg; 0.1 µg/kg to 10µg/kg; 1 µg/kg to 10 mg/kg; 1 µg/kg to 1 mg/kg; 1 µg/kg to 100 µg/kg; 1 µg/kg to 10µg/kg; 10 µg/kg to 10 mg/kg; 10 µg/kg to 1 mg/kg; 10 µg/kg to 100 µg/kg; 100 µg/kg to 10 mg/kg; 100 µg/kg to 1 mg/kg; or 1 mg/kg to 10 mg/kg. Other ranges are contemplated.

In certain embodiments of the present disclosure, the composition comprises the agent in an amount to provide a dose ranging from one of the following selected ranges: 0.01 µg/kg/day to 10 mg/kg/day, 0.01 µg/kg/day to 1 mg/kg/day, 0.01 µg/kg/day to 100 µg/kg/day, 0.01 µg/kg/day to 10 µg/kg/day 0.01 µg/kg/day to 1 µg/kg/day, 0.01 µg/kg/day to 0.1 µg/kg/day, 0.1 µg/kg/day to 10 mg/kg/day; 0.1 µg/kg/day to 1 mg/kg/day; 0.1 µg/kg/day to 100 µg/kg/day; 0.1 µg/kg/day to 10 µg/kg/day; 1 µg/kg/day to 10 mg/kg/day; 1 µg/kg/day to 1 mg/kg/day; 1 µg/kg/day to 100 µg/kg/day; 1 µg/kg/day to 10 µg/kg/day; 10 µg/kg/day to 10 mg/kg/day; 10 µg/kg/day to 1 mg/kg/day; 10 µg/kg/day to 100 µg/kg/day; 100 µg/kg/day to 10 mg/kg/day; 100 µg/kg/day to 1 mg/kg/day; or 1 mg/kg/day to 10 mg/kg/day. Other ranges are contemplated.

In certain embodiments of the present disclosure, the composition comprises the agent in an amount to provide a dose of the agent of less than 100 µg/kg. In certain embodiments of the present disclosure, the composition comprises the agent in an amount to provide a dose of less than 70 µg/kg. In certain embodiments of the present disclosure, the composition comprises the agent in an amount to provide a dose of the agent less than 50 µg/kg. In certain embodiments of the present disclosure, the composition comprises the agent in an amount to provide a dose of the agent less than 25 µg/kg. In certain embodiments of the present disclosure, the composition comprises the agent in an amount to provide a dose of the agent less than 10 µg/kg.

In certain embodiments of the present disclosure, the composition comprises the agent in an amount to provide a dose of the agent of less than 100 µg/kg/day. In certain embodiments of the present disclosure, the composition comprises the agent in an amount to provide a dose of the agent of less than 70 µg/kg/day. In certain embodiments of the present disclosure, the composition comprises the agent in an amount to provide a dose of less than 50 µg/kg/day. In certain embodiments of the present disclosure, the composition comprises the agent in an amount to provide a dose of less than 25 µg/kg/day. In certain embodiments of the present disclosure, the composition comprises the agent in an amount to provide a dose of less than 10 µg/kg/day.

In certain embodiments of the present disclosure, the composition comprises an acceptable carrier suitable for administering the composition to a subject. The carrier may be chosen based on various considerations including the agent(s) being delivered and the time course of delivery of the agents. The term "acceptable carrier" as used herein refers to a substantially inert solid, semi-solid or liquid filler, diluent, excipient, encapsulating material or suitable auxiliary formulation. Physiologically acceptable carriers and their formulations are known in the art.

**In** certain embodiments of the present disclosure, the composition is suitable for administering the agent to the subject on a regular basis, such as daily, twice daily, weekly, monthly, annually or multi-annually administration.

**In** certain embodiments of the present disclosure, the composition is suitable for continuous administration of the agent to the subject.

**In** certain embodiments of the present disclosure, the composition is an immediate release formulation.

**In** certain embodiments of the present disclosure, the composition is a sustained release formulation.

**In** certain embodiments of the present disclosure, the composition provides long-term administration of the agent that reduces activation of the innate immune system. In certain embodiments of the present disclosure, the composition provides long-term continuous administration of the agent that reduces activation of the innate immune system.

In certain embodiments of the present disclosure, the composition comprises a solid substrate with a releasable form of the agent that reduces activation of the innate immune system. Solid substrates suitable for releasing agents are known in the art and are also described herein.

In certain embodiments of the present disclosure, the composition further comprises a sex hormone and/or an agent that modulates production or activity of a sex hormone. Examples of such agents are described herein. A suitable dose may be selected.

In certain embodiments of the present disclosure, the sex hormone comprises one or more of the following: progesterone and its derivatives, dienogest, cyproterone acetate, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17α-ethinyltestosterone and derivatives thereof, 17α-ethinyl-19-nor-testosterone and derivatives thereof, ethynodiol diacetate, dydrogesterone, norethynodrel, allylestrenol, medrogestone, norgestrienone, ethisterone and dl-norgestrel. Other sex hormones are contemplated.

In certain embodiments of the present disclosure, the sex hormone comprises levonorgestrel.

In certain embodiments of the present disclosure, the composition further provides long-term release of the sex hormone and/or the agent that modulates production and/or activity of a sex hormone. In certain embodiments of the present disclosure, the composition further provides long-term continuous release of the sex hormone and/or the agent that modulates production and/or activity of a sex hormone.

In certain embodiments of the present disclosure, the composition comprises a solid substrate with a releasable form of the sex hormone and/or the agent that modulates production and/or activity of a sex hormone.

Certain embodiments of the present disclosure provide a method of treating a female subject suffering from, or susceptible to, a neuroinflammatory condition by intrauterine administration of a composition as described herein.

Certain embodiments of the present disclosure provide use of intrauterine administration of an agent that reduces activation of the innate immune system to treat a female subject suffering from, or susceptible to, a neuroinflammatory condition.

Certain embodiments of the present disclosure provide a product for treating a female subject suffering from, or susceptible to, a neuroinflammatory condition.

In certain embodiments, the present disclosure provides a combination product comprising an agent that reduces activation of the innate immune system. Agents that reduce activation of the innate immune system are described herein.

In certain embodiments, the present disclosure provides a combination product comprising the following components: (i) an agent that reduces activation of the innate immune system; and (ii) a sex hormone and/or an agent that modulates production and/or activity of a sex hormone.

In certain embodiments, the present disclosure provides a combination product comprising the following components: (i) an agent that reduces activation of the innate immune system; and (ii) a sex hormone and/or an agent that modulates production and/or activity of a sex hormone; wherein the components are suitable for separate or combined intrauterine administration to a subject.

In certain embodiments, the present disclosure provides a combination product comprising the following components: (i) a composition suitable for intrauterine and/or vaginal administration as described herein; and (ii) a composition suitable for administration comprising a sex hormone and/or an agent that modulates production and/or activity of a sex hormone. Compositions are described herein.

In certain embodiments of the present disclosure, the components are suitable for separate or combined intrauterine administration to a subject.

Certain embodiments of the present disclosure provide an intrauterine device for treating a female subject suffering from, or susceptible to, a neuroinflammatory.

In certain embodiments, the present disclosure provides an intrauterine device for treating a female subject suffering from, or susceptible to, a neuroinflammatory condition, the device comprising a releasable agent that reduces activation of the innate immune system.

Agents that reduce activation of the innate immune system, and devices providing releasable forms of the agents, are described herein.

In certain embodiments of the present disclosure, the agent reduces activation of a pattern recognition receptor. Pattern recognition modulators and agents that reduce activation of pattern recognition receptors are described herein.

In certain embodiments of the present disclosure, the agent reduces activation of spinal glial cells. Agents that reduce activation of spinal glial cells are described herein.

In certain embodiments of the present disclosure, the agent reduces activation of circulating innate immune cells. Agents that reduce activation of circulating innate immune cells are described herein.

In certain embodiments of the present disclosure, the agent comprises an inhibitor of a Toll-like receptor (TLR), a C-type lectin receptor (CLR), a NOD-like receptor (NDR), a RIG-1 receptor and/or a MDA-5 receptor. Such agents are described herein.

In certain embodiments of the present disclosure, the agent comprises an inhibitor of one of more TLR2, TLR3, TLR4, TLR5, TLR8, TLR9, Dectin-1a, Dectin-1b, Mincle, NOD-1, NOD-2, RIG-1 and MDA-5. Such agents are described herein.

In certain embodiments of the present disclosure, the agent comprises a TLR4 antagonist and/or a TLR2 antagonist.

In certain embodiments of the present disclosure, the agent comprises one or more of amitriptyline, nortriptyline and TAK-242.

In certain embodiments of the present disclosure, the device comprises the agent in an amount to provide a dose ranging from one of the following selected ranges: 0.1 µg/kg to 10 mg/kg; 0.1 µg/kg to 1 mg/kg; 0.1 µg/kg to 100 µg/kg; 0.1 µg/kg to 10µg/kg; 1 µg/kg to 10 mg/kg; 1 µg/kg to 1 mg/kg; 1 µg/kg to 100 µg/kg; 1 µg/kg to 10µg/kg; 10 µg/kg to 10 mg/kg; 10 µg/kg to 1 mg/kg; 10 µg/kg to 100 µg/kg; 100 µg/kg to 10 mg/kg; 100 µg/kg to 1 mg/kg; or 1 mg/kg to 10 mg/kg. Other ranges are contemplated.

In certain embodiments of the present disclosure, the device comprises the agent in an amount to provide a dose ranging from one of the following selected ranges: 0.01 µg/kg/day to 10 mg/kg/day, 0.01 µg/kg/day to 1 mg/kg/day, 0.01 µg/kg/day to 100 µg/kg/day, 0.01 µg/kg/day to 10 µg/kg/day 0.01 µg/kg/day to 1 µg/kg/day, 0.01 µg/kg/day to 0.1 µg/kg/day, 0.1 µg/kg/day to 10 mg/kg/day; 0.1 µg/kg/day to 1 mg/kg/day; 0.1 µg/kg/day to 100 µg/kg/day; 0.1 µg/kg/day to 10 µg/kg/day; 1 µg/kg/day to 10 mg/kg/day; 1 µg/kg/day to 1 mg/kg/day; 1 µg/kg/day to 100 µg/kg/day; 1 µg/kg/day to 10 µg/kg/day; 10 µg/kg/day to 10 mg/kg/day; 10 µg/kg/day to 1 mg/kg/day; 10 µg/kg/day to 100 µg/kg/day; 100 µg/kg/day to 10 mg/kg/day; 100 µg/kg/day to 1 mg/kg/day; or 1 mg/kg/day to 10 mg/kg/day. Other ranges are contemplated.

In certain embodiments of the present disclosure, the device delivers a dose of the agent to the subject of less than 100 µg/kg/day.

In certain embodiments of the present disclosure, the device provides long term continuous release of the agent that reduces activation of the innate immune system.

In certain embodiments of the present disclosure, the device further comprises a releasable sex hormone and/or an agent that modulates production and/or activity of a sex hormone.

In certain embodiments of the present disclosure, the sex hormone comprises levonorgestrel.

In certain embodiments of the present disclosure, the device provides long term continuous release of the sex hormone and/or an agent that modulates production and/or activity of a sex hormone.

Certain embodiments of the present disclosure, the present disclosure provides a method of treating a female subject suffering from, or susceptible to, a neuroinflammatory condition, the method comprising use of an intrauterine device as described herein.

Agents that reduce activation of the innate immune system are described herein.

Devices for intrauterine administration of agents are known in the art. Examples of devices include, for example, devices sold under the brand name "Mirena", "Kyleena" and "Skyla". Devices are described, for example, in Bandyopadhyay A.K. (2008), Novel drug delivery system, 1st edition, Everest publishing house, p. 215-220.

Substrates for use in devices for release of active agents are known in the art, and include for example, copolymers of di-methylsiloxanes and methylvinylsiloxanes, ethylene/vinyl acetate copolymers (EVA), polyethylene, polypropylene, ethylene/propylene copolymers, acrylic acid polymers, ethylene/ethyl acrylate copolymers, polytetrafluoroethylene (PTFE), polyurethanes, thermoplastic polyurethanes and polyurethane elastomers, polybutadiene, polyisoprene, poly(methacrylate), polymethyl methacrylate, styrene-butadiene-styrene block copolymers, poly(hydroxyethyl-methacrylate) (pHEMA), polyvinyl chloride, polyvinyl acetate, polyethers, polyacrylo-nitriles, polyethylene glycols, polymethylpentene, polybutadiene, polyhydroxy alkanoates, poly(lactic acid), poly(glycolic acid), polyanhydrides, polyorthoesters, hydrophilic polymers such as the hydrophilic hydrogels , cross-linked polyvinyl alcohol, neoprene rubber, butyl rubber, hydroxyl-terminated organopolysiloxanes, and copolymers of the aforementioned. Methods for incorporating active agents into a substrate for release are known in the art.

In certain embodiments of the present disclosure, the agent that reduces activation of the innate immune system reduces activation of spinal glial cells and/or reduces activation of circulating innate immune cells. Such agents are described herein.

In certain embodiments of the present disclosure, the agent reduces activation of a pattern recognition receptor. Pattern recognition modulators and agents that reduce activation of pattern recognition receptors are described herein.

In certain embodiments of the present disclosure, the agent comprises an inhibitor of a Toll-like receptor (TLR), a C-type lectin receptor (CLR), and/or a NOD-like receptor (NDR), a RIG-1 receptor and/or a MDA-5 receptor. Agents that inhibit the aforementioned receptors are described herein.

In certain embodiments of the present disclosure, the agent comprises an inhibitor of one of more TLR2, TLR3, TLR4, TLR5, TLR8, TLR9, Dectin-1a, Dectin-1b, Mincle, NOD-1, NOD-2, RIG-1 and MDA-5. Such agents are described herein.

In certain embodiments of the present disclosure, the agent comprises a TLR4 antagonist and/or a TLR2 antagonist.

In certain embodiments of the present disclosure, the agent comprises one or more of amitriptyline, nortriptyline and TAK-242.

In certain embodiments of the present disclosure, the device provides long-term release of the agent that reduces activation of the innate immune system.

In certain embodiments of the present disclosure, the device comprises a membrane controlling release of the agent that reduces activation of the innate immune system.

In certain embodiments of the present disclosure, the device further comprises a releasable sex hormone and/or an agent that modulates production and/or activity of a sex hormone. Sex hormones and/or an agent that modulates production and/or activity of a sex hormone are as described herein.

In certain embodiments of the present disclosure, the sex hormone comprises levonorgestrel.

In certain embodiments of the present disclosure, the device provides long-term release of the sex hormone and/or an agent that modulates production and/or activity of a sex hormone. In certain embodiments of the present disclosure, the device provides long-term continuous release of the sex hormone and/or an agent that modulates production and/or activity of a sex hormone.

Certain embodiments of the present disclosure provide a solid substrate.

In certain embodiments, the present disclosure provides a solid substrate comprising a releasable agent that reduces activation of the innate immune system.

Agents that reduce activation of the innate immune system are described herein.

Substrates suitable for use for releasing active agents are described herein. Amounts of the agents are as described herein.

In certain embodiments, the present disclosure provides a solid substrate comprising releasable amitriptyline. In certain embodiments, the present disclosure provides a solid substrate comprising releasable nortriptyline. In certain embodiments, the present disclosure provides a solid substrate comprising releasable TAK242.

In certain embodiments, the present disclosure provides an intrauterine device for treating a female subject suffering from, or susceptible to, a neuroinflammatory condition, the device comprising a substrate as described herein.

Certain embodiments of the present disclosure provide a method of identifying or screening for new agents for treating a female subject suffering from, or susceptible to, a neuroinflammatory condition.

Neuroinflammatory conditions are described herein.

Agents so identified are potential therapeutic agents for treating a female subject suffering from, or susceptible to, a neuroinflammatory condition.

In certain embodiments, the present disclosure provides a method of identifying an agent for treating a neuroinflammatory condition in a female subject, the method comprising determining the ability of a candidate agent that reduces activation of the innate immune system to treat the neuroinflammatory condition in a female subject by intrauterine administration.

In certain embodiments, the present disclosure provides a method of identifying an agent for treating a neuroinflammatory condition, the method comprising:
(i) providing a candidate agent;
(ii) determining the ability of the candidate agent to reduce activation of the innate immune system; and
(iii) determining the ability of the candidate agent that reduce activation of the innate immune system to treat the neuroinflammatory condition by intrauterine administration.

In certain embodiments of the present disclosure, the neuroinflammatory condition comprises a non-uterine inflammatory pain condition.

In certain embodiments of the present disclosure, the neuroinflammatory condition comprises an inflammatory condition of the central nervous system and/or the peripheral nervous system.

In certain embodiments of the present disclosure, the candidate agent reduces activation of a pattern recognition receptor.

In certain embodiments of the present disclosure, the candidate agent reduces activation of spinal glial cells.

In certain embodiments of the present disclosure, the candidate agent reduces activation of circulating innate immune cells.

In certain embodiments of the present disclosure, the candidate agent is s an inhibitor of a Toll-like receptor (TLR), a C-type lectin receptor (CLR), and/or a NOD-like receptor (NDR), a RIG-1 receptor and/or a MDA-5 receptor.

In certain embodiments of the present disclosure, the candidate agent is an inhibitor of one or more of TLR2, TLR3, TLR4, TLR5, TLR8, TLR9, Dectin-1a, Dectin-1b, Mincle, NOD-1, NOD-2, RIG-1 and MDA-5. Such agents are described herein.

In certain embodiments of the present disclosure, the candidate agent is a TLR4 antagonist and/or a TLR2 antagonist.

In certain embodiments of the present disclosure, the agent is effective when administered intrauterinally at a low dose.

In certain embodiments of the present disclosure, the method comprises *in vitro* screening and or screening in an animal model.

Examples of candidate agents include a drug, a small molecule, a protein, a polypeptide, a lipid, a carbohydrate, a nucleic acid, an oligonucleotide, a ribozyme, a biologic, an aptamer, a cofactor, a ligand, a ligand mimetic, a receptor, a peptidomimetic, an enzyme, a kinase, a phosphatase, a cytokine, a growth factor, a metal ion, a chelate, an antisense nucleic acid, an inhibitor RNA, a microRNA, a siRNA, a sex steroid, an antibody or antigen binding part thereof, an antibody mimetic. Other types of agents are contemplated.

Methods for determining the ability of an agent to reduce activation of the innate immune system are known in the art and described herein.

In certain embodiments, the present disclosure provides an agent identified by the method as described herein.

In certain embodiments, the present disclosure provides a kit for performing a method as described herein.

Agents that reduce activation of the innate immune system are as described herein.

Certain embodiments of the present disclosure provide a method of reducing activation of spinal glial cells in a subject.

Subjects are as described herein.

In certain embodiments of the present disclosure, the present disclosure provides a method of reducing activation of spinal glial cells in a subject, the method comprising intrauterine administration to the subject of an effective amount of an inhibitor of a pattern recognition receptor and thereby reducing activation of the spinal glial cells in the subject.

In certain embodiments of the present disclosure, the method prevents activation of spinal glial cells.

In certain embodiments of the present disclosure, the spinal glial cells comprise astrocytes. In certain embodiments of the present disclosure, the spinal glial cells comprise microglial cells.

Inhibitors of pattern recognition receptors are described herein.

Methods for administration of agents are as described herein. In certain embodiments, the administration comprises use of a device as described herein.

Certain embodiments of the present disclosure provide use of an inhibitor of a pattern recognition receptor for reducing activation of spinal glial cells in a subject.

The present disclosure is further described by the following examples. It is to be understood that the following description is for the purpose of describing particular embodiments only and is not intended to be limiting with respect to the above description.

### EXAMPLE 1 - Glial activation of mice spinal cord by intra-uterine administration of lipopolysaccharide, and effect modification by intra-uterine administration of Toll-Like Receptor modulators

We first recognised that neuroinflammatory conditions of the nervous system may be, at least in part, due to activation of the innate immune system within the uterus resulting in activation of glial cells in the dorsal horn of the spinal cord. To model this in mice, a study was undertaken administering LPS (lipopolysaccharide), a potent immune activator, to the uterus of mice once per estrus cycle over three cycles.

Studies were also undertaken to investigate whether a small dose of amitriptyline, an inhibitor of the pattern recognition receptors TLR, NOD, CLR, RIG-1 and MDA-5, administered with LPS within the uterus could prevent LPS-induced activation of spinal glial cells. To confirm that the amitriptyline was acting, at least in part, by blocking TLRs, an investigation was also conducted on mice given a small dose of pure TLR4 blocker - TAK-242 - with LPS.

The Nonsurgical Embryo Transfer Device (Paratech, USA) was used to allow insertion of fluid into the uterus with brief, mild discomfort to the mouse, and no need for anaesthesia. Following three estrus cycles, glial activation in the dorsal horn of the spinal cord was measured using immunofluorescence. Activation of dorsal horn astrocytes was assessed using GFAP. Activation of dorsal horn microglia was assessed using Iba1. We compared the activation of spinal glia in mice with intrauterine administration of saline only, saline and LPS (100ug/kg), saline LPS(100ug/kg) and Amitriptyline (28ug/kg), and saline LPS (100ug/kg) and TAK-242 (36ug/kg). Representative results are shown in Figure 3, 4 and 5.

The spinal cord was assessed using immunohistochemistry techniques across levels T10, T11, T12, T13, L1, L2, L3, L4, L5, L6, S1 and S2 where T represents the thoracic segment of the spinal cord, L represents the lumbar segment of the spinal cord and S represents the sacral segment of the spinal cord. The regions of interest in the dorsal horn include Laminae I, II, III and IV. Changes in spinal glial reactivity are causally linked to all known models of neuroinflammatory medical conditions.

The results obtained confirmed that intrauterine instillation of intra-uterine LPS induced activation of spinal astrocytes and microglia, as shown in Figure 3.

In addition, intrauterine instillation of amitriptyline with LPS prevented LPS-induced activation of spinal astrocytes and microglia and reduced spinal activation of astrocytes below baseline levels (Figure 4).

We further confirmed that intrauterine instillation of TAK-242 with LPS prevented LPS-induced activation of spinal astrocytes and microglia, and reduced spinal activation of microglia below baseline levels (Figure 5).

The results show that intrauterine LPS induced a substantial increase in GFAP astrocyte staining throughout multiple levels of the spinal cord, when compared with saline controls, and that amitriptyline treatment blocked the impact of intrauterine LPS at multiple levels of the spinal cord and reduced astrocytic reactivity to below basal levels. TAK242 treatment also blocked the impact of intrauterine LPS at multiple levels of the spinal cord and reduced astrocytic reactivity to below basal levels (Figure 8).

Intrauterine LPS induced a localised increase in Iba1 microglial staining throughout select levels of the spinal cord. Amitriptyline treatment blocked the impact of intrauterine LPS at multiple levels of the spinal cord and reduced microglial reactivity to basal levels. TAK242 treatment also blocked the impact of intrauterine LPS at multiple levels of the spinal cord and reduced microglia reactivity to basal levels (Figure 9).

These results demonstrate the ability of intra-uterine LPS to activate spinal glial cells, including astrocytes and microglia. This effect was prevented by the co-administration of amitriptyline. This effect was also prevented by the co-administration of TAK-242 demonstrating that spinal glial activation is mediated, at least in part, by activation of TLR4 receptors. (Figure 10).

Behavioural testing: 24 hours following the final LPS stimulation the Facial Grimace Score was quantified as a proxy marker for pain and compared between groups.: LPS caused a significant increase in the presentation of Grimace behaviours in animals (Figure 6 and Figure 7). Amitriptyline reduced these scores. TAK242 reduced grimace scores non-significantly.

### EXAMPLE 2 - Determination of the innate immune receptors that can reduce spinal glial activation when used within the uterus

The ability of intrauterine TAK-242 to block spinal glial activation following intrauterine administration of LPS confirms the role of TLR4 in this process. Amitriptyline showed additional activity reducing spinal glial activation above TAK-242 alone.

To investigate the range of pattern recognition receptors antagonized by amitriptyline, research was undertaken with 3 aims:
1. To determine the human Toll-Like Receptors which are agonized by lipopolysaccharide (LPS), and thus able to increase activation of glia within the dorsal horn of the spinal cord
2. To determine the human pattern recognition receptors antagonized by amitriptyline (Ami), and thus able to reduce activation of glia within the dorsal horn of the spinal cord. Tested pattern recognition receptors included Toll-Like Receptors 2,3,4,5,7,8, and 9, NOD-Like Receptors (NLR) NOD1 and NOD2, C-Type Lectin Receptor (CLR) (Dectin-1a, Dectin-1b), RIG-I and MDA-5.
3. To confirm the ability of LPS to agonize, and amitriptyline to antagonize TLR4 receptors in human cells.

The range of Toll-Like Receptors antagonized by nortriptyline, a metabolite of amitriptyline, was also investigated.

### Description:

Toll-Like Receptor (TLR), NOD-Like Receptor (NLR) and C-Type Lectin Receptor (CLR) stimulation were tested by assessing NF-κB activation in HEK293 cells expressing a given TLR, NLR or CLR. The activity of the test articles was tested at one concentration and compared to control ligands. These steps were performed in triplicate.

RIG-I and MDA-5 stimulation were tested by assessing IRF3 activation in HEK293 cells expressing human RIG-I or MDA-5 genes. The activity of the test articles was tested on human RIG-I and MDA-5 expressing cells as a potential agonist or antagonist. The test articles were evaluated at one concentration and compared to control ligands. This step was performed in triplicate. The results are provided as Relative Luminescence Units (RLUs).

### Control Ligands:

**TLR2:** HKLM (heat-killed *Listeria monocytogenes*) at 1x10⁸ cells/mL. Antagonist: 1x10⁶ cells/mL:
**hTLR3:** Poly(I:C) HMW at 1 µg/mL Antagonist: 50 ng/mL. **hTLR4:** *E. coli* K12 LPS at 100 ng/mL Antagonist: 10 ng/mL.

**hTLR5:** *S. typhimurium* flagellin at 100 ng/mL Antagonist: 50 ng/mL. **hTLR7:** CL307 at 1 µg/mL Antagonist: 10 ng/mL.

**hTLR8:** CL075 at 1 µg/mL Antagonist: 200 ng/mL.

**hTLR9:** CpG ODN 2006 at 1 µg/mL Antagonist: 500 ng/mL. **hNOD1:** C12-iE-DAP at 1 µg/mL Antagonist: 100 ng/mL.

**hNOD2:** L18-MDP at 100 ng/mL Antagonist: 50 ng/mL.

**hDectin-1a and hDectin-1b:**WGP Soluble (β-glucan from *S. cerevisiae*) at 10 ng/mL Antagonist: 5 ng/mL. Curdlan at 100 µg/mL. Antagonist: 100 µg/mL. Zymosan Depleted (hot alkali treated *S. cerevisiae*) at 5 µg/mL Antagonist: 3 µg/mL.

**hMincle:** Trehalose-6,6-dibehenate (TDB) at 10 µg/mL Antagonist: 5 µg/mL.

**RIG-I/MDA-5:** Poly(I:C)/LyoVec at 1 µg/mL Antagonist: 500 ng/mL. 5'ppp-dsRNA/LyoVec at 1 µg/mL Antagonist: 500 ng/mL. hIFNα at 1000 IU/mL. Antagonist: 100 IU/mL.

RIG-I⁻/MDA-5⁻ Cell Lines: HEK293/Null: Control for human RIG-I and MDA-5 Poly(I:C)HMW/LyoVec at 1 µg/mL. Antagonist: 500 ng/mL 5'ppp-dsRNA/LyoVec at 1 µg/mL. Antagonist: 500ng/mL hIFNα at 1000 IU/mL. Antagonist: 100 IU/mL.

### General Procedure:'

### TLR/NLR/CLR

In a 96-well plate (200 µL total volume) containing the appropriate cells (50,000-75,000 cells/well), 20 µL of the test article or the positive control ligand was added to the wells. For the antagonist assay, the test article was incubated with the cells, at 37°C with 5% CO2, for 30 minutes prior to the addition of the positive control ligand to the wells. The media added to the wells was designed for the detection of NF-κB induced SEAP expression. After a 16-24 hr incubation the optical density (OD) was read at 650 nm on a Molecular Devices SpectraMax 340PC absorbance detector.

### RIG-I/MDA-5

In a 96-well plate (200 µL total volume) containing the appropriate cells (25,000-50,000 cells/well), 20 µL of the test article or of the positive control ligand was added to the wells. For the antagonist assay, the test article was incubated with the cells, at 37°C with 5% CO2, for 30 minutes prior to the addition of the positive control ligand to the wells. After 20 hour incubation, activation of the IRF pathway was monitored using a luciferase detection assay. Luciferase activity was assayed in triplicate from the supernatant of the induced cells, and the relative luminescence units (RLUs) are detected by a Promega GloMax Luminometer.

### Results:

### Human TLR and NLR Agonist Screening:

The fold induction (ratio of average induced value to average non-induced value) results were as follows:

| **TLR/NLR Cell Line** | **Control-** | **LPS-EB 20 ng/mL** | **Control+** |
|---|---|---|---|
| hTLR2 | 1 | 10 | 17 |
| hTLR3 | 1 | 1 | 17 |
| hTLR4(MD2-CD14) | 1 | 14 | 16 |
| hTLR5 | 1 | 1 | 18 |
| hTLR7 | 1 | 1 | 34 |
| hTLR8 | 1 | 1 | 19 |
| hTLR9 | 1 | 1 | 18 |
| hNOD 1 | 1 | 1 | 20 |
| hNOD2 | 1 | 1 | 10 |

**Human TLR/NLR Cell lines:** hTLR2: HKLM (heat-killed *Listeria monocytogenes* 1x10⁸cells/mL . **hTLR3:** Poly(I:C) HMW at 1 µg/mL. **hTLR4 (MD2-CD14):** *E. coli* K12 LPS at 100 ng/mL. **hTLR5:** *S. typhimurium* flagellin at100 ng/mL . **hTLR7:** CL307 at 1 µg/mL. **hTLR8:** CL075 at 1 µg/mL. **hTLR9:** CpG ODN 2006 at 1 µg/mL **hNOD1**: C12-iE-DAP at 1 µg/mL **hNOD2:** L18-MDP at100ng/mL

### Human CLR, RLR, Agonist Screening:

### Fold Induction (Ratio of average induced value to average non-induced value):

| **CLR Cell Line** | **Control ⁻** | **LPS-EB 20 ng/mL** | **WGP Soluble 10 ng/mL** | **Curdlan 100 µg/mL** | **Zymosan Depleted 5 µg/mL** |
|---|---|---|---|---|---|
| hDectin-la | 1 | 1 | 14 | 6 | 15 |
| hDectin-1b | 1 | 1 | 1 | 8 | 26 |

### RIG-I

| **Control ⁻** | **LPS-EB 20 ng/mL** | **Poly(I:C)/LyoVec 1 µg/mL** | **5'ppp-dsRNA/LyoVec 1 µg/mL** | **hIFNα 1000 IU/mL** |
|---|---|---|---|---|
| 1 | 1 | 11 | 31 | 169 |

### MDA-5

| **Control ⁻** | **LPS-EB 20 ng/mL** | **Poly(I:C)/LyoVec 1 µg/mL** | **5'ppp-dsRNA/LyoVec 1 µg/mL** | **hIFNα 1000 IU/mL** |
|---|---|---|---|---|
| 1 | 1 | 5 | 1 | 2 |
| | | | | 5 |
| | | | | 2 |

### Human TLR Antagonist Screening:

Fold induction (ratio of average induced value to average non-induced value) was determined for each TLR, NOD1, NOD2, Dectin-1a, Dectin-1b.

| Human TLR2 | | **+ HKLM 1x10^6 cells/mL** | | |
|---|---|---|---|---|
| | **Control ⁻** | **Ami 1 µM** | **Nor 1 µM** | **No Sample** |
| Fold Induction* | 1 | 5 | 5 | 6 |

| Human TLR3 | | **+ Poly(I:C) 50 ng/ml** | | |
|---|---|---|---|---|
| | **Control ⁻** | **Ami 1 µM** | **Nor 1 µM** | **No Sample** |
| Fold Induction* | 1 | 12 | 12 | 14 |

| Human TLR4 | | + **LPS-EK 10 ng/mL** | | |
|---|---|---|---|---|
| | **Control ⁻** | **Ami 1 µM** | **Nor 1 µM** | **No Sample** |
| Fold Induction* | 1 | 12 | 14 | 14 |

| Human TLR5 | | **+ FLA-ST 50 ng/ml** | | |
|---|---|---|---|---|
| | **Control ⁻** | **Ami 1 µM** | **Nor 1 µM** | **NoSample** |
| Fold Induction* | 1 | 33 | 34 | 34 |

| Human TLR7 | | **+ CL307 10 ng/ml** | | |
|---|---|---|---|---|
| | **Control ⁻** | **Ami 1 µM** | **Nor 1 µM** | **No Sample** |
| Fold Induction* | 1 | 16 | 16 | 13 |

| Human TLR8 | | **+ CL075 200 ng/ml** | | |
|---|---|---|---|---|
| | **Control ⁻** | **Ami 1 µM** | **Nor 1 µM** | **No Sample** |
| Fold Induction* | 1 | 11 | 11 | 13 |

| Human TLR9 | | + **ODN 2006 500 ng/ml** | | |
|---|---|---|---|---|
| | **Control ⁻** | **Ami 1 µM** | **Nor 1 µM** | **No Sample** |
| Fold Induction* | 1 | 6 | 6 | 7 |

| Human NOD1 | | **+ C12-iE-DAP 100 ng/mL** | |
|---|---|---|---|
| | **Control ⁻** | **Ami 1 µM** | **No Sample** |
| Fold Induction* | 1 | 9 | 10 |
| | | | |

| Human NOD2 | | + **L18-MDP 50 ng/mL** | |
|---|---|---|---|
| | **Control ⁻** | **Ami 1 µM** | **No Sample** |
| Fold Induction* | 1 | 17 | 18 |

| Human Dectin-la | | **+ WGP Soluble 5 ng/mL** | | **+ Curdlan 100 µg/mL** | | **+ Zymosan Depleted 3 µg/mL** | |
|---|---|---|---|---|---|---|---|
| | **Control** ⁻ | **Ami 1 µM** | **No Sample** | **Ami 1 µM** | **No Sample** | **Ami 1 µM** | **No Sample** |
| Fold Induction* | 1 | 8 | 8 | 4 | 5 | 4 | 6 |

| Human Dectin-1b | | **+ WGP Soluble 5 ng/mL** | | **+ Curdlan 100 µg/mL** | | **+ Zymosan Depleted 3 µg/mL** | |
|---|---|---|---|---|---|---|---|
| | **Control** - | **Ami 1 µM** | **No Sample** | **Ami 1 µM** | **No Sample** | **Ami 1 µM** | **No Sample** |
| Fold Induction* | 1 | 1 | 1 | 4 | 4 | 6 | 7 |

| Human Mincle | | **+ TDB 5 µg/mL** | |
|---|---|---|---|
| | **Control ⁻** | **Ami 1 µM** | **No Sample** |
| Fold Induction* | 1 | 5 | 6 |

### Human RLR Antagonist Screening:

### Fold induction (ratio of average induced value to average non-induced value).

### RIG-I

| | **Poly(I:C)/LyoVec 500 ng/mL** | | **5'ppp-dsRNA/LyoVec 500 ng/mL** | | **hIFNα 100 IU/mL** | |
|---|---|---|---|---|---|---|
| **Control**⁻ | **Ami 1 µM** | **No Sample** | **Ami 1 µM** | **No Sample** | **Ami1 µM** | **No Sample** |
| 1 | 16 | 16 | 6 | 6 | 82 | 108 |

### MDA-5

| | **Poly(I:C)/LyoVec 500 ng/mL** | | **5'ppp-dsRNA/LyoVec 500 ng/mL** | | **hIFNα 100 IU/mL** | |
|---|---|---|---|---|---|---|
| **Control ⁻** | **Ami 1 µM** | **No Sample** | **Ami 1 µM** | **No Sample** | **Ami 1 µM** | **No Sample** |
| 1 | 10 | 9 | 1 | 1 | 192 | 255 |

### Summary of Results:

### Human TLR/NLR

Test Article LPS-EB exhibits a significant stimulatory effect on human TLR2 and TLR4.

Test Article Amitriptyline exhibits a slight inhibitory effect on human TLR2, 3, 4, 5, 8, 9, NOD1 and NOD2 stimulated with their respective positive control ligands. In addition, Amitriptyline exhibits a slight inhibitory effect on TLR⁻/NLR⁻ control cell lines HEK293/Null1 and HEK293/Null2 when stimulated with TNFa at 10 ng/mL. The inhibitory effect observed on the negative control cell lines indicates that the inhibitory effect observed on human TLR2, 3, 4, 5, 8, 9, NOD1 and NOD2 may not be due to TLR/NLR specific inhibition. Test article Amitriptyline exhibits a slight potentiating effect on human TLR7.

Test Article Nortriptyline exhibits a slight inhibitory effect on human TLR2, 3, 8 and 9 stimulated with their respective positive control ligands. In addition, Nortriptyline exhibits a slight inhibitory effect on TLR⁻/NLR⁻ control cell lines HEK293/Null1 and HEK293/Null2 when stimulated with TNFa at 10 ng/mL. The inhibitory effect observed on the negative control cell lines indicates that the inhibitory effect observed on humanTLR2, 3, 8 and 9 may not be due to TLR specific inhibition. Test article Nortriptyline exhibits a slight potentiating effect on human TLR7.

### Human CLR

Test Article LPS-EB does not exhibit a stimulatory effect on human Dectin-1a, or Dectin-1b or Mincle.

Test Article Amitriptyline exhibits a slight inhibitory effect on human Dectin-1a when stimulated with Curdlan at 100 µg/mL and Zymosan Depleted at 3 µg/mL. In addition, Amitriptyline exhibits a slight inhibitory effect on human Dectin-1b when stimulated with Zymosan Depleted at 3 µg/mL. Amitriptyline also exhibits a slight inhibitory effect on human Mincle when stimulated by TDB at 5 µg/mL.

### Human RLR

Test Article LPS-EB does not exhibit a stimulatory effect on human RIG-I or MDA-5.

Test Article Amitriptyline exhibits an inhibitory effect on human RIG-I, MDA-5 and RIG-I⁻/MDA-5⁻ control cell line HEK293/Null when stimulated with recombinant hIFNα at 100 IU/mL. The inhibitory effect observed on the negative control cell line (and only on recombinant hIFNα) indicates that the antagonistic effect observed is not due to specific RIG-I or MDA5 inhibition.

### Conclusion:

In summary, the testing has shown that amitriptyline and nortriptyline provide broad inhibition of TLR, CLR and RLR receptors, and are likely to provide a beneficial effect to reduce immune activation within the uterus.

### EXAMPLE 3 - Increased responsiveness of circulating innate immune cells to TLR4 stimulation associated with increased symptoms in a group of young women

Increased activation of circulating PBMCs is associated with a reduced EC₅₀ for IL-1β release following the *in vitro* stimulation of cells with the TLR agonist LPS.

Patient recruitment: 55 women aged between 16 and 35 years were recruited. A questionnaire was completed asking about the presence or absence of subjective symptoms that may be experienced by women with activation of the innate immune system. A blood sample was taken on Day 7-10 of their menstrual cycle. Blood samples were subjected to the following laboratory assessment to determine whether evidence of activation of circulating innate immune cells could be found.

Laboratory method: Isolated PBMCs were diluted to 1 x 10⁶ cells·mL⁻¹ in enriched RPMI 1640 (10% (v/v) fetal calf serum and 1% (v/v) penicillin), and plated into 96 well plates (Nunc, Roskilde, Denmark) using 100µL per well. Sufficient cells were obtained from all participants, and plasma was not collected. As no reference range for LPS dose is available for this group, TLR responsiveness was assessed across a range of concentrations. Triplicate wells were treated with a dosage curve of TLR agonist LPS; 13pg·mL⁻¹ - 10µg·mL⁻¹ (Sigma-Aldrich, Castle Hill, NSW, Australia). Control wells contained no LPS. Plates were incubated for 20 hours at 37 °C and 5% CO₂ in a humidified environment (Thermoline Scientific, Sydney, Australia). IL-1β levels were determined using a commercially available ELISA kit (IL-1β ELISA; BD Bioscience, Australia) according to the manufacturer's instructions. The absorbance was quantified on a BMG Polarstar microplate reader (BMG Labtechnologies, Offenburg, Germany) at 450nm. The manufacturer's limit of quantification of 0.8pg·mL⁻¹ was used, with any readings below this removed from further analysis.

Using statistical modelling, the EC₅₀ for each participant was determined using the following method:
Concentration - response curves following LPS stimulation were fitted to an Eₘₐₓ model using the Hill equation using a non-linear mixed-effects model approach. The slope parameter was fixed to 1 to reduce the number of parameters to be estimated. The model used was of the form:
$E = {E}_{0} + \frac{{E}_{max} * C}{{EC}_{50} + C}$
where E₀ is the response Y at baseline (absence of dose), Eₘₐₓ is the asymptotic maximum dose effect (maximum effect attributable to the drug), and EC₅₀ is the concentration which produces 50% of the maximal effect. Results are displayed in Table1.

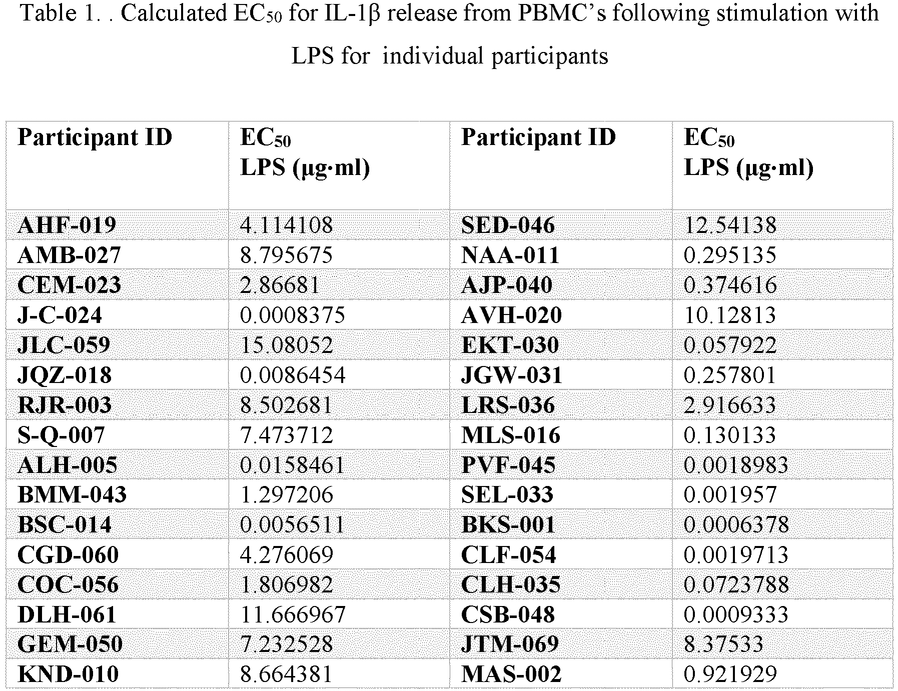

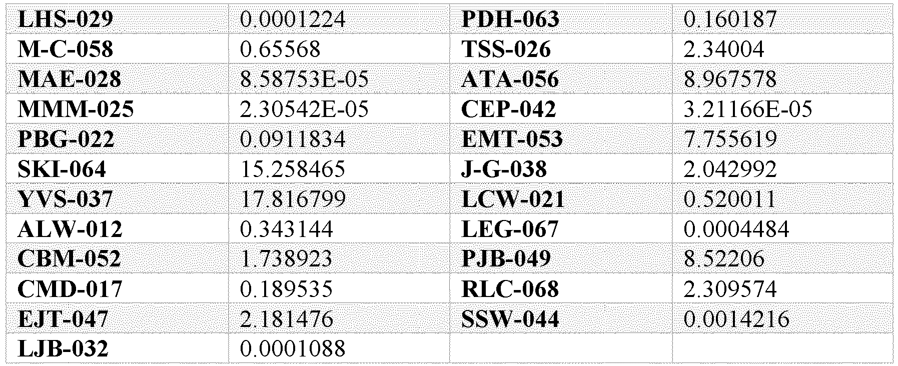

Further investigation used an unpaired, two-tailed, parametric t test to compare the EC₅₀ for IL-1β release (Mean ± SEM) from PBMC's following LPS stimulation in women with and without symptoms commonly associated with neuroinflammatory conditions and found a significantly lower EC₅₀ for the human symptoms of diarrhoea, bloating, constipation, headaches, anxiety, low mood and poor sleep on Day 7-10 of the menstrual cycle of as shown in Table 3. These results provide further evidence for a link between the activation of circulating immune cells and human symptoms that are commonly associated with neuroinflammatory conditions affecting the nervous system. The significant association between these symptoms and the EC₅₀ at a time outside menstruation removes the presence of menstruation as a confounding factor.

**Table 2. Symptoms significantly associated with a reduced EC₅₀ for IL-1β release from PBMC's following stimulation with LPS in young women**

| Symptoms reported | EC₅₀ |
|---|---|
| | Day 7-10 |
| | * p value<0.01 |
| | **p value<0.05 |
| Bowel symptoms | 0.026** |
| Diarrhoea | 0.098* |
| Bloating | 0.014 ** |
| Constipation | 0.041** |
| Headaches | 0.037** |
| Anxiety | 0.039 ** |
| Depression | 0.038 * |
| Poor sleep | 0.028** |

In connection with these studies, the considerations set out below provide further support for the present disclosure.
- The severity of premenstrual symptoms is associated with raised blood cytokine inflammatory markers, with those women with the highest levels reporting the most severe symptoms, suggesting a role for activation of the innate immune system.
- Circulating innate immune cells have access to the brain emphasized by the recently discovered missing link between the brain and the body, the lymphatic vessels of the brain, providing an opportunity for peripheral inflammation to influence central nervous system function.
- Circulating cells of the innate immune system may be activated by stimulation of Toll-Like Receptors (TLRs), and that TLRs are widely expressed in the body including within the uterine endometrium, the spinal cord and the brain.
- Stimulation of uterine endometrium with lipopolysaccharide, a TLR4 agonist, results in inflammation within the uterine cavity, activation of the innate immune system, and the secretion of cytokines.
- *Esherichia Coli,* a bacteria with TLR4 agonist lipopolysaccharide within its cell wall, is present in the uterine cavity of a proportion of women. This provides an opportunity for the chronic activation of the innate immune system.
- Oral medications whose mechanisms of action include the inhibition of TLRs, have been used to treat these disorders. For example, amitriptyline, a tricyclic medication and TLR inhibitor, is used orally in clinical practice for the prevention of migraine, and has been used successfully to treat PMS and depression. However, oral amitriptyline may be associated with adverse effects including sedation, which may be unacceptable to a subject.

Accordingly, targeting pattern recognition activity within the uterus, with a low dose of inhibitor offers two potential novel therapeutic mechanisms for the reduction of non-uterine symptoms in a subject, with reduced potential for adverse effects.

The inhibition of pattern recognition activity within the uterus may result in reduced activation of the innate immune system, resulting in a reduction in activated circulating innate immune cells and associated cytokines, and a reduction of non-uterine symptoms in the subject.

The inhibition of TLR activity within the uterus has been demonstrated to result in a reduction of glial cell activation (these studies) within the dorsal horn of the spinal cord. This provides an additional mechanism for the reduction of symptoms within a subject.

### EXAMPLE 4 - An intrauterine device for treating a neuroinflammatory condition in a female subject

A patient suitable for treatment suffering from a neuropsychiatric condition, may be treated with an intrauterine device providing long-term release of an agent that reduces activation of the innate immune system.

For example, a patient may be treated with an intrauterine device providing long-term release of an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2, RIG-1 or MDA-5.

The intrauterine device provides a delivery system for long-term release of an agent that inhibits a Toll-like receptor 4 (TLR4), thereby administering the active agent to the patient.

Intrauterine devices for administering active agents are known in the art, for example as described in US Patent Application No. 20140127280.

For example, the intrauterine device may utilise a body construction with a core reservoir comprising a TLR4 antagonist, and optionally a membrane encasing the core, made from a suitable polymer. The polymer may be selected on the desired release rates of the active agents.

The polymer composition of the core and/or the membrane can be chosen so that the intrauterine system releases a sufficient predetermined amount of a TLR4 antagonist.

A membrane may cover the whole or part of the reservoir to further control the release rate of the active agent(s). The polymer composition used in the membrane is such that it allows the pre-determined, constant release rates of the active agent(s). The composition and/or thickness of the membrane may be selected upon the desired release profile of the active agent(s), and may have one or more layers.

The polymer in the core and/or the membrane is generally selected to have high biocompatibility.

The release kinetics of an active agent from a polymer based delivery system depends on a variety of characteristics such as the molecular weight, solubility, diffusivity and charge of the therapeutically active agent, as well as on the characteristics of the polymer, the loading of the therapeutically active agent, the distance the therapeutically active agent must diffuse through the device to reach its surface and on the characteristics of any matrix or membrane.

Polysiloxanes, such as poly(dimethyl siloxane) (PDMS), may be to regulate the release rate of active agents. Polysiloxanes are physiologically inert, and a wide group of active agents are capable of penetrating polysiloxane membranes, which also suitable strength properties. The release rate of active agent(s) can be adjusted as a desired by modifying the polymeric material in a suitable way, e.g. by adjusting hydrophilic or hydrophobic properties of the material. For example, addition of poly (ethylene oxide) groups or trifluoropropyl groups to a PDMS polymer may change the release rate of active agents.

Further examples of suitable materials include, copolymers of di-methylsiloxanes and methylvinylsiloxanes, ethylene/vinyl acetate copolymers (EVA), polyethylene, polypropylene, ethylene/propylene copolymers, acrylic acid polymers, ethylene/ethyl acrylate copolymers, polytetrafluoroethylene (PTFE), polyurethanes, thermoplastic polyurethanes and polyurethane elastomers, polybutadiene, polyisoprene, poly(methacrylate), polymethyl methacrylate, styrene-butadiene-styrene block copolymers, poly(hydroxyethyl-methacrylate) (pHEMA), polyvinyl chloride, polyvinyl acetate, polyethers, polyacrylo-nitriles, polyethylene glycols, polymethylpentene, polybutadiene, polyhydroxy alkanoates, poly(lactic acid), poly(glycolic acid), polyanhydrides, polyorthoesters, hydrophilic polymers such as the hydrophilic hydrogels, cross-linked polyvinyl alcohol, neoprene rubber, butyl rubber, hydroxyl-terminated organopolysiloxanes, and copolymers of the aforementioned.

The core or the membrane may also comprise additional materials to further adjust the release rate of the active agent(s), for example complex forming agents such as cyclodextrin derivatives to adjust an initial release of the active agent to the accepted or desired level.

In one embodiment, the core and the membrane are made of a siloxane based elastomer composition comprising at least one elastomer and optionally a non-crosslinked polymer. Siloxane-based elastomers include elastomers made of poly (disubstituted siloxanes) where the substituents mainly are lower alkyl, preferably alkyl groups of 1 to 6 carbon atoms, or phenyl groups, wherein the alkyl or phenyl can be substituted or unsubstituted. For example, poly(dimethylsiloxane) (PDMS) may be used.

Examples of elastomeric compositions include an elastomer composition comprising poly(dimethylsiloxane) (PDMS), an elastomer composition comprising a siloxane-based elastomer comprising 3,3,3-trifluoropropyl groups attached to the silicon atoms of the siloxane units, an elastomer composition comprising poly(alkylene oxide) groups, the poly(alkylene oxide) groups being present as alkoxy-terminated grafts or blocks linked to the polysiloxane units by silicon-carbon bonds or as a mixture of these forms, and one or more combinations of the above.

In one example, the siloxane-based elastomer comprises from 1 to approximately 50% of the substituents attached to the silicon atoms of the siloxane units as 3,3,3-trifluoropropyl groups. The percentage of the substituents that are 3,3,3-trifluoropropyl groups can be for example 5-40%, 10-35%, 1-29% or 15-49.5%.

In another example, the siloxane-based elastomer comprises poly(alkylene oxide) groups so that the poly(alkylene oxide) groups are present in the elastomer either as alkoxy-terminated grafts of polysiloxane units or as blocks, said grafts or blocks being linked to the polysiloxane units by silicon-carbon bonds. For example, poly(alkylene oxide) groups such as poly(ethylene oxide) (PEO) groups may be used.

Methods for the preparation of suitable polymers are provided, for example, in international patent applications WO 00/00550, WO 00/29464, WO 99/10412 and US Patent Application No. 20140127280.

Agents that inhibit Toll-like receptor 4 (TLR4) are described herein.

The intrauterine device may also release other therapeutic agents, such as a sex hormone. For example, the sex hormone may be a progestogenic compound. Examples of progestogenic compounds include compounds such as progesterone and its derivatives, cyproterone acetate, dienogest, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17α-ethinyltestosterone and derivatives thereof, 17α-ethinyl-19-nor-testosterone and derivatives thereof, ethynodiol diacetate, dydrogesterone, norethynodrel, allylestrenol, medrogestone, norgestrienone, ethisterone and dl-norgestrel.

The release of the active agent(s) may be selected to occur over a suitable period of time, for example from weeks to years.

The amount of the agent that inhibits Toll-like receptor 4 (TLR4) incorporated in the delivery system varies depending on the particular active agent and the time for which the intrauterine delivery system is expected to provide therapy.

The shape and size of the intrauterine device may be chosen by a person skilled in the art compatible with the dimensions of the uterine cavity. For example, an intrauterine delivery system may comprise a body forming the frame of the system and a reservoir containing the active agent(s) attached on the body. One example is a T-shaped object fabricated of a biocompatible material and having an elongate member having at one end a transverse member comprising two arms, the elongate member and the transverse member forming a T-shaped piece when the system is positioned in the uterus. Reservoirs containing an active agent(s) can be attached to the elongate member, to the transverse member or members, or both to the elongate member and the transverse member(s). The manufacturing of such devices is known in the art.

For example, the body and the reservoir may be manufactured simultaneously or separately followed by their assembly. The body may be manufactured by injection moulding or compression moulding. The active agent containing cores may be manufactured by mixing the therapeutically active substance or substances within the core matrix material for example such as polydimethylsiloxane (PDMS, processed to the desired shape by moulding, casting, extrusion, or by other appropriate methods known in the art.

A membrane layer, if any, can be applied onto the core according to known methods such as by using extrusion or injection moulding methods, spraying or dipping. As an alternative, a prefabricated membrane tube may be expanded mechanically for example with a suitable device or by using for example pressurized gas, such as air, or by swelling it in a suitable solvent, such as cyclohexane, diglyme, isopropanol, or in a mixture of solvents, where after the swollen membrane tube is mounted onto the core. When the solvent evaporates, the membrane tightens on the core.

A reservoir containing the active agent(s) may be fixed on the frame by using a variety of different methods. The frame may for example comprise an elongated extension in the form of a polymer shaft, core, rod or pin or the like at a suitable point on which the hollow tube-like reservoir is assembled, for example by first enlarging the diameter of the reservoir tube to some degree, for example by using pressure or solvent swelling, and thereafter by simply sliding the reservoir onto the extension or inserting the extension into the hollow reservoir. It is also possible to assemble first the hollow tube-like core onto the body and then assemble the membrane onto the core. Other methods to attach the reservoir to the frame include for example known techniques of welding, use of an adhesive, or use of special metal or polymer inserts, clips, connectors, adapters, clothespin-type means or clamps.

If needed, one or each end of the reservoirs so obtained may be sealed by using known techniques, for example by applying a drop of an adhesive or silicon glue.

The intrauterine delivery system can also be manufactured by coating the body with the drug containing core material by using known technology, for example such as dipping, spraying, injection moulding and like.

The core can also be prepared for example by using a coextrusion method described in the Finnish patent FI 97947. The active agent(s) may be mixed within the core matrix polymer composition, and processed to the desired shape and size by using known extrusion methods.

The body of the system may further comprise locking means to keep the cores or reservoir in place during the insertion step, during the use of the device or during the removal of the device.

The delivery system can be manufactured in any size as required, the exact size being dependent on the patients and particular application. In practice, the dimensions of the delivery system should be close to the size of the uterine cavity. For a human female, the length of the IUS body is normally in the order of from 20 to 40 mm. in length, preferably from 25 to 38 mm and the width of the body is in the order of from 20 to 32 mm corresponding generally to the width of the fundal portion of the uterine cavity. The cross-sectional diameter of the body member is in the order of from 1 to 4 mm, preferably from 1.5 to 3 mm.

The length of the core of the delivery system will be chosen to give the required performance. The length of the reservoir as well as of a core segment can be for example from 1 to 35 mm and depends on the nature of the material.

The outer diameter of the core can be, for example, from 0.1 to 5.0 mm, and preferably from 0.2 to 3.5 mm. The thickness of the membrane encasing the core or core segment may be, for example, from 0.1 to 1.0 mm, preferably from 0.2 to 0.6 mm.

For example, 45 parts by weight of a TLR4 antagonist, levonorgestrel, 50 parts by weight of poly(dimethylsiloxane-co-vinylmethylsiloxane) and 1.2 parts by weight of di-chlorobenzoylperoxide-polydimethylsiloxane paste (50% of dichlorobenzoylperoxide) may be mixed with a 2-roll mill. The mixture may be extruded to a tube-like form with a wall thickness of 0.8 mm and outer diameter of 2.8 mm and cured by heat for 15 minutes, during which crosslinking will take place. The crosslinked core may then be cut into 24 mm length.

In an alternative example, 54 parts of commercial poly(dimethylsiloxane-co-vinylmethylsiloxane), 45.5 parts by weight of a TLR4 antagonist, 0.4 parts of poly(hydrogenmethylsiloxane-co-dimethylsiloxane) crosslinker, 0.02 parts of ethynyl cyclohexanol inhibitor and 10 ppm of Pt-catalyst (of the reaction species) in vinyl-methylsiloxane may be mixed in a kneading mill. The mixture may be extruded to a tube-like form with a wall thickness of 0.7 mm and cured by heat for 30 minutes and cooled.

The core may then be swollen in cyclohexane and pulled over the IUS body and cyclohexane allowed to evaporate.

The release rate of the agent that inhibits Toll-like receptor 4 (TLR4) from the implant may be determined *in vitro.* The intrauterine delivery system may be placed in a dissolution medium shaken at a suitable speed at 37° C. The dissolution medium may be withdrawn and replaced by a fresh dissolution medium at predetermined time intervals, and the amount of the released active agent analysed by using standard analytical methods.

Examples of a suitable amount of a TLR4 antagonist for incorporation into the device for release may be in the range from 270 mg to 540 mg, which represents an amount of the agent suitable for 6 months to 1 year of treatment for a patent of approximately 60 kg body weight.

The insertion of the intrauterine device may occur using standard procedures. Efficacy of the treatment may be evaluated by assessing a variety of suitable clinical parameters, such as described in Example 4.

It will also be appreciated that the device described above may have other uses, for example in one or more of the embodiments described herein.

### EXAMPLE 5 - Intrauterine administration of an agent that reduces activation of the innate immune system can reduce premenstrual syndrome (PMS) in women

A study may be undertaken to assess the ability of an agent that reduces activation of the innate immune system to reduce the symptoms of PMS in women.

For example, a double-blinded study may be undertaken to assess the ability of an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, to reduce the symptoms of PMS in women.

Following selection of subjects suffering the symptoms of PMS, each woman will be subject to a daily intrauterine composition comprising either a low dose of the agent (eg 1.7 mg amitriptyline) or placebo. PMS symptoms will be measured using daily self-reported symptom scales in both groups.

Methods for producing intrauterine compositions are known in the art. Compositions containing either a low dose of the agent or vehicle only will be sourced from a compounding pharmacy. Compositions will be prepared, randomised and numbered by the pharmacist with master code kept in a locked facility by the pharmacist at the compounding laboratory.

Outcome measures will be assessed by symptom score, quality of life questionnaire, and patient satisfaction with the composition. It is anticipated that intrauterine administration of Amitriptyline will reduce premenstrual symptoms in women.

### EXAMPLE 6 - Intrauterine administration of an agent that reduced activation of the innate immune system can reduce the frequency of migraine in women

A study may be undertaken to assess the ability of an agent that reduces intrauterine activation of the innate immune system to reduce the frequency or severity of migraines in women.

For example, a double-blinded study may be undertaken to assess the ability of an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, to reduce the frequency or severity of migraines in women.

Women with migraines will be subject to a daily uterine composition comprising either a low dose of the agent (eg 1.7 mg amitriptyline) or placebo.

Patient outcomes will be measured by self-reported pain and symptom scales, and by assessing the analgesic requirement for both groups. Outcome measures will be assessed by VAS pain score, symptom score and quality of life questionnaire.

It is anticipated that intrauterine administration of the agent will reduce migraine frequency, or severity in women.

### EXAMPLE 7 - Intrauterine administration of an agent that reduces activation of the innate immune system can reduce the exacerbation of psychological or psychiatric symptoms in women

A study may be undertaken to assess the ability of an agent that reduces intrauterine activation of the innate immune system to reduce exacerbations of psychological or psychiatric conditions in women.

For example, a double-blinded study may be undertaken to assess the ability of an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, administered as a uterine composition to reduce exacerbations of psychological or psychiatric symptoms in women. Women reporting exacerbation of their symptoms at this time will be subject to a daily uterine composition comprising either a low dose of the agent (eg 1.7 mg amitriptyline) or placebo.

Patient outcomes will be measured by self-reported symptom scales. Outcome measures will be assessed by symptom score and quality of life questionnaire.

It is anticipated that intrauterine administration of the agent will reduce or prevent the exacerbation of the psychological or psychiatric symptoms.

### EXAMPLE 8 - Enhanced treatment of a woman with premenstrual syndrome or premenstrual dysphoric disorder

The medical conditions PMS and PMDD, as described in the DSM-V occurs exclusively in the luteal phase of the menstrual cycle. In this example, a woman suffering from PMS or PMDD and requests treatment of her symptoms may be selected.

For example, an intrauterine device comprising an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, will be inserted in the uterus. An example of such a device is described above and also shown in Figure 13. Methods for producing a device are described herein.

It is anticipated that the device will reduce the severity of PMS or PMDD symptoms.

### EXAMPLE 9 - Enhanced treatment of a woman with migraine

Migraine headaches are more common in women than men. In this example, a woman with migraines may be selected. An intrauterine device comprising an agent that an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, may be inserted in the uterus.

For example, an intrauterine device comprising the agent will be inserted in the uterus. An example of such a device is described herein and also shown in Figure 13. Methods for producing a device are described herein.

It is anticipated that the device will reduce the severity of migraines in the luteal phase of her menstrual cycle.

### EXAMPLE 9 - Enhanced treatment of a woman with established psychological or psychiatric disorder, such as depression or anxiety, who reports an exacerbation of symptoms in the luteal phase of the menstrual cycle.

Certain psychological or psychiatric disorders are may be exacerbated in the luteal phase of the menstrual cycle. In this example, a woman suffering from an established psychological or psychiatric condition, such as depression or anxiety, who reports an exacerbation of symptoms in the luteal phase of the menstrual cycle, and requests treatment of her premenstrual symptoms, may be selected.

For example, an intrauterine device comprising an agent that reduces activation of the innate immune system, and which amitriptyline inhibits, such as TLR2, TLR3, TLR4, TLR5, TLR8, TLR9, Dectin-1a, Dectin-1b, Mincle, NOD1, NOD2 RIG-1 or MDA-5, will be inserted in the uterus. An example of such a device is described above and also shown in Figure 13. Methods for producing a device are described herein.

It is anticipated that the device will reduce the exacerbation of her psychological or psychiatric symptoms in the luteal phase of the menstrual cycle.

### FURTHER REFERENCES

Barton GM, Kagan JC (2009) Nat. Rev. Immunol. 9(8), 535-42;
Blasius AL, Beutler B (2010) Immunity 32(3), 305-15;
Bao et al. (2018) Int. J. Pharm. 550(1-2): 447-453;
Kawai T, Akira S (2010) Nat. Immunol. 11(5), 373-84;
Lester SN, Li K (2014) J. Mol. Biol. 426(6), 1246-64;
Li X, Jiang S, Tapping RI (2010) Cytokine 49(1), 1-9;
McGettrick AF, O'Neill LA (2010) Curr. Opin. Immunol. 22(1), 20-7;
Miggin SM, O'Neill LA (2006) J. Leukoc. Biol. 80(2), 220-6; Pasare C, Medzhitov R (2005) Adv. Exp. Med. Biol. 560, 11-8;
Reuven EM, Fink A, Shai Y (2014) Biochim. Biophys. Acta 1838(6), 1586-93
Coats SR. et al. (2005). J Immunol. 175(7):4490-8;
Wang et al (2013) Chem Soc Rev. 42(12): 4859-4866;
Cheng, K., et al. 2012. Angew. Chem. Int. Ed. 51, 12246;
Sahoo et al (2013) American Journal of Advanced Drug Delivery: ISSN-2321-547X;
Bhowmik et al (2010) Annals of Biological Research 1(1): 70-75;
Widermeesch D. (2010) Hand. Exp Pharmacol. 197: 268-298
Bandyopadhyay A.K. (2008), Novel drug delivery systems, 1st edition; Everest publishing house, p. 215-220;
Remington's Pharmaceutical Sciences, 17th ed., Mack Publishing Company, Easton, Pa., 1985;
Langford et al (2010) Nature Methods 7: 447-449
US Patent Application No. 20140127280;
WO 00/00550;
WO 00/29464;
WO 99/10412; and
Finnish patent FI 97947.
Diagnostic and Statistical Manual of Mental Disorders (5th Edition). Arlington, VA: American Psychiatric Association. 2013. p. 625.4. Code: 625.4 (N94.3).

Also, it is to be noted that, as used herein, the singular forms "a", "an" and "the" include plural aspects unless the context already dictates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Reference to any prior art in this specification is not, and should not be taken as, an acknowledgment or any form of suggestion that this prior art forms part of the common general knowledge in any country.

The subject headings used herein are included only for the ease of reference of the reader and should not be used to limit the subject matter found throughout the disclosure or the claims. The subject headings should not be used in construing the scope of the claims or the claim limitations.

The description provided herein is in relation to several embodiments which may share common characteristics and features. It is to be understood that one or more features of one embodiment may be combinable with one or more features of the other embodiments. In addition, a single feature or combination of features of the embodiments may constitute additional embodiments.

The methods described herein can be performed in one or more suitable orders unless indicated otherwise herein or clearly contradicted by context. The use of examples, or exemplary language (e.g., "such as") provided herein, is intended merely to better illuminate the example embodiments and does not pose a limitation on the scope of the claimed invention unless otherwise claimed. No language in the specification should be construed as indicating any non-claimed element as essential.

## Claims

1. An agent that reduces activation of the innate immune system for use in a method of treating a female subject suffering from, or susceptible to, a neuroinflammatory condition, the method comprising intrauterine administration to the subject of an effective amount of the agent that reduces activation of the innate immune system and thereby treating the subject, wherein the agent is amitriptyline, wherein the neuroinflammatory condition is premenstrual syndrome (PMS) or premenstrual dysphoric disorder (PMDD), wherein the treatment improves or reduces an affective symptom and/or behavioural symptom in the subject, said symptom being selected from one or more mood swings, tearfulness, sensitivity to rejection, irritability or anger, marked depressed mood, hopelessness, self-deprecating thoughts, anxiety, tension difficulty concentrating and a sense of feeling overwhelmed.

2. The agent for use according to claim 1, wherein the agent reduces activation of spinal glial cells.

3. The agent for use according to claim 1 or 2, wherein the agent reduces activation of spinal glial astrocyte cells.

4. The agent for use according to any one of claims 1 to 3, wherein the agent reduces activation of circulating innate immune cells.

5. The agent for use according to any one of claims 1 to 4, wherein the administration of amitriptyline to the subject comprises administration a dose of less than 100 µg/kg/day.

6. The agent for use according to any one of claims 1 to 5, wherein the administration of the agent comprises long term continuous administration to the subject.

7. The agent for use according to any one of claims 1 to 6, wherein the method further comprises administration of a sex hormone and/or an agent that modulates production and/or activity of a sex hormone.

8. The agent for use according to any one of claims 1 to 7, wherein the intrauterine administration of the agent comprises release of the agent from an intrauterine device.

## Patentansprüche

1. Mittel, das die Aktivierung des angeborenen Immunsystems verringert, zur Verwendung in einem Verfahren zur Behandlung einer weiblichen Patientin, die an einem neuroentzündlichen Zustand leidet oder dafür anfällig ist, wobei das Verfahren die intrauterine Verabreichung einer wirksamen Menge des Mittels, das die Aktivierung des angeborenen Immunsystems verringert, an die Patientin und damit die Behandlung der Patientin umfasst, wobei das Mittel Amitriptylin ist, wobei der neuroentzündliche Zustand prämenstruelles Syndrom (PMS) oder prämenstruelle dysphorische Störung (PMDD) ist, wobei die Behandlung ein affektives Symptom und/oder ein Verhaltenssymptom bei der Patientin verbessert oder verringert, wobei das Symptom aus einem oder mehreren ausgewählt ist von Stimmungsschwankungen, Weinerlichkeit, Empfindlichkeit gegenüber Ablehnung, Reizbarkeit oder Wut, ausgeprägter depressiver Stimmung, Hoffnungslosigkeit, selbstabwertenden Gedanken, Angst, Anspannung, Konzentrationsschwierigkeiten und einem Gefühl der Überforderung.

2. Mittel zur Verwendung gemäß Anspruch 1, wobei das Mittel die Aktivierung von spinalen Gliazellen verringert.

3. Mittel zur Verwendung gemäß Anspruch 1 oder 2, wobei das Mittel die Aktivierung von spinalen Glia-Astrozyten verringert.

4. Mittel zur Verwendung gemäß einem der Ansprüche 1 bis 3, wobei das Mittel die Aktivierung von zirkulierenden Zellen des angeborenen Immunsystems verringert.

5. Mittel zur Verwendung gemäß einem der Ansprüche 1 bis 4, wobei die Verabreichung von Amitriptylin an die Patientin die Verabreichung einer Dosis von weniger als 100 µg/kg/Tag umfasst.

6. Mittel zur Verwendung gemäß einem der Ansprüche 1 bis 5, wobei die Verabreichung des Mittels eine langfristige, kontinuierliche Verabreichung an die Patientin umfasst.

7. Mittel zur Verwendung gemäß einem der Ansprüche 1 bis 6, wobei das Verfahren ferner die Verabreichung eines Sexualhormons und/oder eines Mittels umfasst, das die Produktion und/oder die Aktivität eines Sexualhormons moduliert.

8. Mittel zur Verwendung gemäß einem der Ansprüche 1 bis 7, wobei die intrauterine Verabreichung des Mittels die Freisetzung des Mittels aus einer intrauterinen Vorrichtung umfasst.

## Revendications

1. Agent réduisant l'activation du système immunitaire inné, destiné à être utilisé dans un procédé de traitement d'un sujet féminin souffrant d'une affection neuroinflammatoire ou susceptible d'en souffrir, le procédé comprenant l'administration intra-utérine au sujet d'une quantité efficace de l'agent réduisant l'activation du système immunitaire inné et, par conséquent, le traitement du sujet, dans lequel l'agent est l'amitriptyline, dans lequel l'affection neuroinflammatoire est le syndrome prémenstruel (SPM) ou le trouble dysphorique prémenstruel (TDPM), dans lequel le traitement améliore ou réduit un symptôme affectif et/ou un symptôme comportemental chez le sujet, ledit symptôme étant choisi parmi un ou plusieurs parmi les sautes d'humeur, la tendance à pleurer, la sensibilité au rejet, l'irritabilité ou la colère, l'humeur dépressive marquée, le désespoir, les pensées d'autodévalorisation, l'anxiété, la tension, la difficulté à se concentrer et le sentiment d'être dépassé.

2. Agent destiné à être utilisé selon la revendication 1, dans lequel l'agent réduit l'activation de cellules gliales spinales.

3. Agent destiné à être utilisé selon l'une des revendications 1 ou 2, dans lequel l'agent réduit l'activation de cellules astrocytaires gliales spinales.

4. Agent destiné à être utilisé selon l'une quelconque des revendications 1 à 3, dans lequel l'agent réduit l'activation de cellules immunitaires innées circulantes.

5. Agent destiné à être utilisé selon l'une quelconque des revendications 1 à 4, dans lequel l'administration d'amitriptyline au sujet comprend l'administration d'une dose inférieure à 100 µg/kg/jour.

6. Agent destiné à être utilisé selon l'une quelconque des revendications 1 à 5, dans lequel l'administration de l'agent comprend une administration continue à long terme au sujet.

7. Agent destiné à être utilisé selon l'une quelconque des revendications 1 à 6, dans lequel le procédé comprend en outre l'administration d'une hormone sexuelle et/ou d'un agent qui module la production et/ou l'activité d'une hormone sexuelle.

8. Agent destiné à être utilisé selon l'une quelconque des revendications 1 à 7, dans lequel l'administration intra-utérine de l'agent comprend la libération de l'agent à partir d'un dispositif intra-utérin.
